# EUROPEAN PATENT APPLICATION

(11) **EP 2 377 917 A1**
(43) Date of publication of application: **19.10.2011**
(21) Application number: 09837469.7
(22) Date of filing: 28.12.2009
(51) Int. Cl.: C12M 1/00, C12N 15/09

(54) **MICROINJECTION APPARATUS AND MICROINJECTION METHOD**

(30) Priority: 09.01.2009 JP 2009003599; 22.01.2009 JP 2009011884; 08.04.2009 JP 2009093599; 26.08.2009 JP 2009194965
(71) Applicant: NTN Corporation, Osaka-shi, Osaka 550-0003 (JP)
(72) Inventor: OZAKI, Takayoshi, Iwata-shi Shizuoka 438-0037 (JP); YAMADA, Hiroyuki, Iwata-shi Shizuoka 438-0037 (JP); SUZUKI, Kenichi, Iwata-shi Shizuoka 438-0037 (JP); MARUI, Naoki, Iwata-shi Shizuoka 438-0037 (JP)
(74) Representative: Baumgärtel, Gunnar
(86) International application number: PCT/JP2009/007328
(87) International publication number: WO 2010/079580

(57) **Abstract**

A microinjection apparatus is designed to introduce the transfecting material into the transductant by inserting a minute injection needle (11) into the transductant and includes a container position adjuster (1), an imaging device (2), a position determiner (3) and a plurality of transporters (4). The container position adjuster adjusts the position of a container (12) accommodating therein the transductant. The imaging device captures through a lens (2a), an image of an inside of the container, which has been adjusted in position by the container position adjuster. The position determiner determines the position of the transductant from the image obtained by the imaging device. The plurality of the transporters are operable to transport the plural injection needles, individually for each of those injection needles, in dependence on the position information obtained by the position determiner.

## Description

### CROSS REFERENCE TO THE RELATED APPLICATION

This application is based on and claims Convention priorities to Japanese patent applications No. 2009-003599, filed January 9, 2009, No. 2009-011884, filed January 22, 2009, No. 2009-093599, filed April 8, 2009, and No. 2009-194965, filed August 26, 2009, the entire disclosures of all of which are herein incorporated by reference as a part of this application.

### BACKGROUND OF THE INVENTION

### (Field of the Invention)

The present invention relates to a microinjection apparatus for and a microinjection method of injecting a transfecting material such as, for example, a gene regulatory factor into a transductant such as, for example, a cell, into which such material is to be introduced, with the use of a fine injection needle.

### (Description of Related Art)

As an exemplary method of introducing a desired substance such as, for example, a gene regulatory factor into a cell, the electroporation method which is an electrical system, the lipofection method which is a chemical system, the vector method which is a biological system, the microinjection method which is a mechanical system, and the laser injection method which is an optical system are currently available.

Of them, the electroporation method which is the electric system is apt to tear the cell membrane by means of high current and the cell is therefore damaged considerably. In the case of the lipofection method which is the chemical system, the gene regulatory factor that can be introduced as the transfecting material is limited and, therefore, the transfection efficiency is low. Even in the case of the vector method which is the biological system, the gene regulatory factor that can be introduced is limited and, also, a problem is found in security. In the case of the microinjection method which is the mechanical system, there is such an advantage that a highly accurate control of the injection position makes it possible to accursedly introduce the transfecting material into the cell. As one example of this microinjection method, a method has been suggested, in which with the use of a capillary (a minute injection needle) the transfecting material is introduced into the cell (See the Patent Document 1 listed below.).

As a technique to increase the processing efficiency of the microinjection method, the method has been suggested, in which a microcapillary array, in which a plurality of injection needles are regularily arranged, and a microchamber array, in which a plurality of microchambers are arranged to fix cells at respective positions corresponding to the injection needles of the microcapillary array are disposed in face-to-face relation with each other so that the gene regulatory factor can be injected into a plurality of cells at a time.

### (See the Patent Document 2 listed below.)

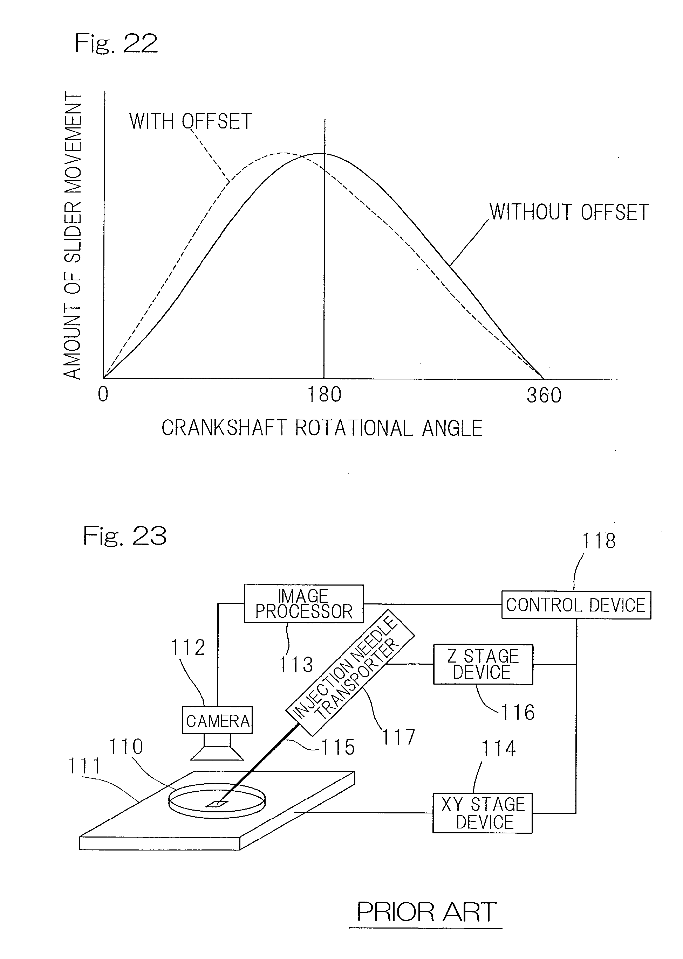

One example of the conventional apparatus to which the microinjection method is applied is shown in Fig. 23. In this illustrated microinjection apparatus, a position information of the cell is obtained by holding a petri dish 130 accommodating therein a cell, which is a target material or a transductant, with a container support table 131, then capturing a local plan view image of an inside of the petri dish 130 with an imaging device 132, and finally processing the plan view image so captured with an image processor 133. When the container support table 131 is moved by means of a container position adjuster 134 of a horizontal biaxial direction comprised of an XY stage device, the cell can be positioned so that the cell can assume a position conforming to the direction of injection of an injection needle 135. Then, a manipulator holding the injection needle 135 is moved to the direction of injection of the injection needle 135 by means of an injection needle transporter 137 including a Z-axis stage device 136. Thus, the injection needle 135 is pierced into the cell so positioned and the transfecting material filled inside the injection needle 135 is introduced into the cell. A series of those operations are automatically performed by the control of a control device 138. For the injection needle transporter 137, an ultrasonic motor or a ball screw is employed.

### [Prior Art Literature]

[Patent Document 1] JP Patent No. 2624719
[Patent Document 2] JP Patent No. 3035608

While the foregoing methods have been enumerated as a method of introducing a gene regulatory factor such as, for example, DNA or protein into a cell, no technique have been established, which strikes a balance between reliability and efficiency. Of those known methods, however, the microinjection method of introducing a gene regulatory factor into each of cells appears to be the most reliable method, but there is a problem that the operation thereof requires a substantial amount of skill and time and, for this reason, the efficiency is low.

In the method such as, for example, the microcapillary array system disclosed in the Patent Document 2, in which respective positions of the injection needles relative to the plurality of the cells are controlled all at a time, the injection needle tends to fail to piece into most of the cells or most of the cells tend to be damaged. Also, in the microcapillary array system, since the cells are required to be arranged at the same position as the injection needles in the microcapillary array, it is merely applicable only to suspended cells movable within a culture fluid and cannot be applied to the cells deposited on the bottom of the petri dish or the culture medium.

In view of the foregoing, in an apparatus used to perform the microinjection method, attempts have hitherto been made to increase the injecting speed at which the transfecting material is injected into the cell and also to increase the positioning speed at which the manipulator is repositioned, in order to increase the processing efficiency thereof. The microinjection apparatus currently placed in the market is available, which the injection process is accomplished at a rate shorter than 1 second in injecting the transfecting material into the single cell.

However, according to the recent demands arising in the field of medical applications, requirements have been made that the apparatus can handle any transfecting material to be injected into the cell of any kind, the transfection efficiency is high and a large amount of the cells, into which the transfecting material has been introduced, can be supplied, and particularly the important requirement that the large amount of the cells can be supplied has not yet been fulfilled.

Also, even though a plurality of injection needles are used, the manipulator equipped with the injection needles is currently large in size and, therefore, there is a problem that a plurality of the manipulators cannot be arranged.

In addition, in the currently available microinjection apparatus shown in and described with reference to Fig. 23, for example, since the container position adjuster 134 supporting the petri dish 130 accommodating the cell for positioning the cell is moved so that the manipulator equipped with the injection needle 135 can be driven in a direction only towards the direction in which the injection needle 135 is pierced into the cell, positioning of a plurality of the cells to allow the injection needle 135 to be pierced into all of those cells simultaneously has been impossible to achieve.

### SUMMARY OF THE INVENTION

An object of the present invention is to provide a microinjection apparatus in which introduction of the transfecting material into the transductant such as, for example, the cell can be assuredly and efficiently accomplished with a plurality of injection needles to thereby increase the processing efficiency.

Another object of the present invention is to enable the simultaneous positioning of respective tip ends of the plural injection needles relative to the plural transductants to thereby further increase the processing efficiency.

The microinjection apparatus of the present invention is a microinjection apparatus for introducing an transfecting material into a transductant by inserting a minute injection needle into the transductant, the injection needle being filled with the transfecting material therein, which apparatus includes a container position adjuster for adjusting a position of a container accommodating therein the transductant; a plurality of transporters for individually moving a plurality of injection needles to the transductant within the corresponding container, of which position has been adjusted by the container position adjuster; an imaging device for capturing through a magnifying lens, an image of an inside of the container, of which position has been adjusted by the container position adjuster; a position determiner for determining a position of the transductant from the image obtained by the imaging device; and a controller for causing each of the transporters to move the injection needle in dependence on a position information obtained by the position determiner.

According to the above described construction, by adjusting the position of the container, accommodating therein the transductant, by means of the container position adjuster, capturing the plan view image of the inside of the container having been adjusted in position, and finally determining the cell position from the image so captured by means of the position determiner, the transductant such as, for example, the cell, which the respective injection needle has to be assigned, is determined. At this time, by locally magnifying the container with a magnifying lens and then image processing the position of the transductant such as, for example, the cell, the position of the transductant can be recognized accurately. Also, the transductant assigned by each of the transporters can be determined from, for example, the positional relation within the image of the transductant such as, for example, the cell that has been recognized, In this way, by inserting the injection needle into the transductant with the injection needles having been moved individually by the plurality of the transporters after the transductant which the respective injection needle is to be assigned has been determined, the transfecting material is introduced into each of those transductants. Because of this, introduction of the transfecting material into the pural transductants such as, for example, the cells can be accomplished simultaneously and the efficiency of the injection processing can be increased. Also, by filling injecting materials of at least two or more kinds in the injection needles or the like and successively introducing them into the same transductant, simultaneous introduction of those transfecting materials of the plural kinds into the transductant can be accomplished. Accordingly, with no need to supersede the material injected into the injection needle, the transfecting materials of the plural kinds can be introduced and the efficiency of the injection processing can be increased.

In the present invention, the plurality of the transporters may be arranged in a radial pattern around the container. Since in this microinjection apparatus, positioning of each of the injection needles is carried out by the individual transporter, arrangement of the plural transporters in the radial pattern around the container as hereinabove described above can be made possible. Where the plural transporters for positioning the injection needles are arranged in the radial pattern around the container, a number of transporters can be arranged in a limited space around the container and, hence, a number of the injection needles can be used and, therefore, the efficiency of the injection processing can further be increased.

In the present invention, the controller may be so configured as to recognize respective positions of a plurality of transductants within the container according to the position information obtained by the position determiner and as to actuate the plurality of the transporter parallel to insert a plurality of injection needles into the plural transductants.

When the injection operation is carried out by actuating the plural transporters, which are operable to actuate the individual injection needles, parallel, the efficiency of the injection processing can be increased. While the plural transporters are parallel actuated, unlike the positioning of the plural injection needles with a common transporter, since the individual transporters are capable of positioning independently, a proper positioning to the plural transductants such as, for example, the cells can be accomplished, enabling an assured injection and increasing the injection processing.

In the present invention, the controller is so configured as to recognize a position of the transductant within the container according to the position information obtained by the position determiner and as to successively actuate the plurality of the transporters to insert a plurality of the injection needles into the same transductant.

In the case of the above described construction, in the injection processing during which the plural types of transfecting materials are injected with the plural injection needles, respectively, increase of the efficiency is possible.

In the present invention, the microinjection apparatus may include a transporter retracting mechanism for advancing or retracting each of the transporters between a ready-to-inject position, at which a tip end of the injection needle approaches the transductant, and a retracted position.

While the tip end of each of the injection needles may be held at the ready-to-inject position proximate to the transductant at all times, in such case, the injection needle will constitute an obstruction at the time of replacement of the container accommodating therein the transductant, making it difficult to achieve a smooth replacement of the container. The replacement of the container can be smoothly and readily accomplished when the transporter retracting mechanism is employed. Also, unlike the apparatus in which the range of movement of each of the transporters is expanded to allow the injection needle to be retracted from the container, the transporters may be sufficient if it can move within a small range such as, for example, within the field of view of the imaging device and as a result, retraction of the injection needle from the container can be accomplished while an attempt is being made to downsize the transporter.

In the present invention, the transductant may be a cell and the transfecting material may be a gene regulatory factor. The gene regulatory factor is, for example, DNA or protein. Where the transductant is a cell and the transfecting material is a gene regulatory factor, the effect, described above, that the processing efficiency can be increased as a result of the capability of accomplishing a simultaneous introduction of the transfecting material into the plural transductants can be more effectively exhibited.

In the present invention, each of the transporters may have at least two degrees of freedom and the transfer mechanisms are provided one for each degree of freedom, and in which case at least one of the transfer mechanisms has a drive source employed in the form of a piezoelectric elements laminate comprised of a plurality of piezoelectric elements laminated and capable of expanding or contracting in a direction of lamination thereof.

According to the above described construction, since particularly the transporter has at least two degrees of freedom with respective transfer mechanisms and at least one of the transfer mechanisms has a drive source employed in the form of a piezoelectric elements laminate comprised of a plurality of piezoelectric elements laminated, the transporter can have a reduced size and the plurality of the transporters can be arranged in a multi-stage in a limited space around the container and, hence, the plurality of the injection needles can be employed, thus making it possible to increase the efficiency of the injection processing.

In the present invention, the transfer mechanism in each of the transporters, the drive source of which is employed in the form of the piezoelectric elements laminate, may be of a structure in which a plurality of piezoelectric elements laminates are arranged parallel to each other and connected in series with each other in a direction conforming to expansion or contraction thereof by means of a fastening member.

Where the plurality of the piezoelectric elements laminates are arranged parallel to each other and connected in series with each other, a further large displacement can be obtained with a compact structure. In order for the tip end of the injection needle to be movable within the entire region of the predetermined field of view in the image obtained by the imaging device, the distance of movement of the injection needle caused by the transporter is preferably not smaller than 1 mm, but arranging and connecting the piezoelectric elements laminates in the manner described above is effective to secure a sufficient amount of movement required by the injection needle without any large space being required.

In the present invention, the transfer mechanism in each of the transporters, the drive source of which is employed in the form of the piezoelectric elements laminate, may include an amplifying mechanism for amplifying a displacement of the expansion or contraction of the piezoelectric elements laminate in a direction perpendicular to a direction of expansion or contraction of the piezoelectric elements laminate. In the case of the above described construction, it is more effective to secure the amount of movement over which the injection needle is required to move.

The amplifying mechanism may include a link mechanism that is made up of first, second and third links, one fixed joint fixed in position and first, second and third movable joints each movable in position and is operable to convert the displacement of the piezoelectric elements laminate in the direction of expansion or contraction to a transverse direction and also to amplify such displacement.

According to the above described construction, because of the link mechanism, the direction of displacement can be converted with a simplified structure and such displacement can be considerably amplified. Each of the joints referred to above may be a coupling for coupling component parts that are connected with each other through a rolling bearing or a slide bearing. Where the rolling bearing is used, rattling can be reduced when the frictional resistance is reduced and a proper preload is applied to the rolling bearing, thus enabling a precise positioning. Also, since there is no elastically deformable portion, designing of the amplifying mechanism is easy to achieve. Each of the joints referred to above is a pivotable joint forming a nodal point and having a center of pivot lying perpendicular to any one of the direction of expansion and contraction of the piezoelectric elements laminate and the direction perpendicular to the direction of expansion and contraction. The first link having a base end connected with the fixed joint has the other end connected with an intermediate portion of the second link, having a base end connected with the first movable joint, through the second movable joint; the third link is connected with a tip end of the second link through the third movable joint; the third link has a tip end rendered to be a movable segment which is constrained for movement only in the direction perpendicular to expansion and contraction. The fixed joint referred to above is fixed in position relative to an end of the piezoelectric elements laminate on a fixed side; the first movable joint is made movable together with an end of the piezoelectric elements laminate on an expansion side; and the movable segment at the tip end of the third link is rendered to be a displacement amplifying output unit of the link mechanism.

In the case of the above described construction, there is no need to connect the three links at the same position and to juxtapose the two joints in the axial direction and, therefore, the thicknesswise dimension of the link mechanism can be reduced.

The amplifying mechanism may include a link mechanism that is made up of first and second links, a fixed joint fixed in position and first and second movable joints movable in position and is operable to convert the displacement of the piezoelectric elements laminate in the direction of expansion or contraction to a transverse direction and also to amplify such displacement.

In the construction described above, each of those joints referred to above is a pivotable joint forming a nodal point and in which the first link having a base end connected with the fixed joint has the other end connected with an intermediate portion of the second link, which has a base end connected with the first movable joint, through the second movable joint. The first link having a base end connected with the fixed joint has the other end connected with an intermediate portion of the second link, which has a base end connected with the first movable joint, through the second movable joint; and in which the fixed joint is fixed in position relative to an end of the piezoelectric elements laminate on a fixed side; the first movable joint is rendered to be movable together with an end of the piezoelectric elements laminate on an expansion side. The second link has a tip end which will become a displacement amplifying unit of the link mechanism.

Even in the case of the construction described above, there is no need to connect the three links at the same position and to juxtapose the two joints in the axial direction and, therefore, the thicknesswise dimension of the link mechanism can be reduced.

In the present invention, the transfer mechanism in each of the transporters, the drive source of which is employed in the form of the piezoelectric elements laminate, may include a first amplifying mechanism for amplifying the expansion or contraction of the piezoelectric element into a displacement in a direction perpendicular to the direction of expansion or contraction and a second amplifying mechanism for amplifying the displacement, which has been amplified by the first amplifying mechanism, into a displacement in the direction of expansion or contraction of the piezoelectric element. In the case of the construction described above, it is further effective to secure the required amount of movement over which the injection needle is required to move.

Each of the first and second amplifying mechanisms may include a link mechanism that is made up of first, second and third links, a fixed joint fixed in position and first, second and third movable joints each movable in position, in which case each of the link mechanisms forming the respective first and second amplifying mechanism may be combined in two stage relative to each other so that the displacement can be successively transmitted.

Each of those joints is a pivotable joint forming a nodal point and having a center of pivot lying perpendicular to any one of the direction of expansion and contraction of the piezoelectric elements laminate and the direction perpendicular to the direction of expansion and contraction. The first link having a base end connected with the fixed joint has the other end connected with an intermediate portion of the second link, having a base end connected with the first movable joint, through the second movable joint; the third link is connected with a tip end of the second link through the third movable joint; the third link has a tip end rendered to be a movable segment which is constrained for movement only in the direction perpendicular to expansion and contraction.

The fixed joint in the link mechanism forming the first amplifying mechanism is fixed in position relative to an end of the piezoelectric elements laminate on a fixed side; and the first movable joint is made movable together with an end of the piezoelectric elements laminate on an expansion side.

The link mechanism forming the second amplifying mechanism is provided in such an attitude that the link mechanism forming the first amplifying mechanism is altered 90° about a center axis parallel to an axis of pivot of each of the joints; the fixed joint is fixed in position relative to an end of the piezoelectric elements laminate on a fixed side and the first movable joint is provided in the movable segment in the link mechanism forming the first amplifying mechanism. The movable segment of the third movable joint in the link mechanism forming the second amplifying mechanism is rendered to be a displacement amplifying output unit of the link mechanism.

Each of the first and second amplifying mechanisms may include a link mechanism that is made up of first and second links, a fixed joint fixed in position and first and second movable joints movable in position, in which case each of the link mechanisms forming the respective first and second amplifying mechanism may be combined in two stage relative to each other so that the displacement can be successively transmitted.

Each of those joints is a pivotable joint forming a nodal point and has a center of pivot lying perpendicular to any one of the direction of expansion and contraction of the piezoelectric elements laminate and the direction perpendicular to the direction of expansion and contraction. Each of the link mechanism is of a structure in which the first link having a base end connected with the fixed joint has the other end connected with an intermediate portion of the second link, which has a base end connected with the first movable joint, through the second movable joint.

The fixed joint in the link mechanism forming the first amplifying mechanism is fixed in position relative to an end of the piezoelectric elements laminate on a fixed side; the first movable joint is rendered to be movable together with an end of the piezoelectric elements laminate on an expansion side.

The link mechanism forming the second amplifying mechanism is provided in such an attitude that the link mechanism forming the first amplifying mechanism is altered 90° about a center axis parallel to an axis of pivot of each of the joints; the fixed joint is fixed in position relative to an end of the piezoelectric elements laminate on a fixed side and the first movable joint is provided in a tip end of the second link in the link mechanism forming the first amplifying mechanism. The tip end of the second link in the link mechanism forming the second amplifying mechanism is rendered to be a displacement amplifying output unit of the link mechanism.

The amplifying means may include a link mechanism and in which the link mechanism includes a cranking and sliding mechanism comprised of one fixed joint, two movable joint and two links and being capable of amplifying and converting a displacement of the piezoelectric elements in a direction of expansion or contraction thereof into an arbitrary direction along a circumference of the fixed joint through the two movable joints and the two links.

In the construction described above, the fixed joint is arranged, for example, on the same line in the direction of movement of the first movable joint. In this structure, each of the joints is a pivotable joint forming a nodal point and has a center of pivot lying perpendicular to any one of the direction of expansion and contraction of the piezoelectric elements laminate and the direction perpendicular to the direction of expansion and contraction. By setting the output unit at an arbitrary position on the circumference of the fixed joint, the displacement can be amplified in an arbitrary direction. Accordingly, reduction in number of component parts and reduction in size can be accomplished.

Also, although the cranking and sliding mechanism is of the structure in which the fixed joint is arranged on the same line in the direction of movement of the movable joint, it may be an offset cranking mechanism in which the fixed joint does not lie on the same line in the direction of movement of the movable joint. In this case, because of the offset, the angle of rotation of the fixed joint relative to the reciprocal movement of the first movable joint varies for each stroke of reciprocal movement. Accordingly, by selecting the point, at which the angle of rotation of the fixed joint is large, relative to the amount of movement of the first movable joint, the displacement amplifying rate can be increased with no need to increase the dimension of the link.

In the present invention, the transfer mechanism for moving a needle support member, supporting the injection needle, in a direction conforming to a direction of insertion of the injection needle, may be a transfer mechanism including the piezoelectric elements laminate as a drive source and may include a vibration driver for applying vibration to the needle support member in a direction conforming to the direction of insertion of the injection needle by vibrating a part of piezoelectric elements forming the transfer mechanism.

In the case of the above described construction, not only can the injection needle be smoothly pierced into the transductant, but also a high speed drive can be enabled for positioning the injection needle.

The microinjection method of the present invention is a microinjection method of introducing a transfecting material into a transductant by piercing a minute injection needle, filled with the transfecting material, into the transductant, which method includes a container position adjusting step of adjusting a position of a container accommodating therein the transductant; an imaging step of capturing a plan view image of an inside of the container, the position of which has been adjusted during the container position adjusting step, through a lens; a position determining step of determining a position of the transductant from the image obtained during the imaging step; and a step of individually moving each of a plurality of injection needles by means of a plurality of transporters according to a position information obtained during the position determining step.

According to this microinjection method, in a manner similar to that described above in connection with the microinjection apparatus, not only the simultaneous introduction of the transfecting material into the plurality of the transductants such as, for example, cells, but also successive introduction of the plurality of the transporters into the same transductant can be accomplished one at a time and, therefore, the efficiency of the injection processing can be increased advantageously.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 is a conceptual diagram showing a microinjection apparatus according to a first preferred embodiment of the present invention;
Fig. 2 is a top plan view showing an arrangement of transporter employed in the microinjection apparatus;
Fig. 3 is a perspective view showing an assembly of a transporter retracting mechanism with one of the transporter in the microinjection apparatus;
Fig. 4 is a longitudinal sectional view showing a Z-axis transfer mechanism employed in the transporter;
Fig. 5 is a horizontal sectional view showing a Y-axis transfer mechanism employed in the transporter;
Fig. 6 is a horizontal sectional view showing an X-axis transfer mechanism employed in the transporter;
Fig. 7 is a perspective view showing a container support platform employed in the microinjection apparatus;
Fig. 8 is a sectional view showing one example of a cell adsorbing substrate unit used to fix a suspended cell;
Fig. 9 is a top plan view showing an X-Y stage device employed in a container position adjuster;
Fig. 10 is a fragmentary perspective view, with a portion cut out, showing one of the transporter employed in the microinjection apparatus according to a second preferred embodiment of the present invention;
Fig. 11 is a diagram showing in combination a longitudinal sectional view, illustrating one example of the Z-axis transfer mechanism employed in the transporter, and a block diagram, illustrating a conceptual structure of a control circuit thereof;
Fig. 12 is a diagram showing in combination a longitudinal sectional view, illustrating another example of the Z-axis transfer mechanism employed in the transporter, and a block diagram, illustrating a conceptual structure of a control circuit thereof;
Fig. 13 is a diagram showing in combination a horiontal sectional view, illustrating the Y-axis transfer mechanism employed in the transporter, and a block diagram, illustrating a conceptual structure of a control circuit thereof;
Fig. 14 is a diagram showing in combination a horizontal sectional view, illustrating the X-axis transfer mechanism employed in the transporter, and a block diagram, illustrating a conceptual structure of a control circuit thereof;
Fig. 15 is a diagram showing in combination a horizontal sectional view, illustrating another example of the Y-axis transfer mechanism employed in the transporter, and a block diagram, illustrating a conceptual structure of a control circuit thereof;
Fig. 16A is a top plan view showing an example of the structure of a link mechanism employed in the Y-axis transfer mechanism;
Fig. 16B is a top plan view, showing another example of the structure of the link mechanism employed in the Y-axis transfer mechanism, and a fragmentary enlarged sectional view thereof;
Fig. 16C is a top plan view showing another example of the structure of the link mechanism employed in the Y-axis transfer mechanism;
Fig. 17 is a diagram showing in combination a horizontal sectional view, illustrating the X-axis transfer mechanism employed in the transporter, and a block diagram, illustrating a conceptual structure of a control circuit thereof;
Fig. 18 is a top plan view showing another example of the structure of the link mechanism employed in the X-axis transfer mechanism in the transporter;
Fig. 19A is a diagram showing in combination a horizontal sectional view, illustrating another example of the structure of the Y-axis transfer mechanism employed in the transporter, and a block diagram, illustrating a conceptual structure of a control circuit thereof;
Fig. 19B is a diagram showing in combination a horizontal sectional view, illustrating another example of the structure of the X-axis transfer mechanism employed in the transporter, and a block diagram, illustrating a conceptual structure of a control circuit thereof;
Fig. 20 is a top plan view showing one example of the structure of a link mechanism of the Y-axis transfer mechanism and the X-axis transfer mechanism;
Fig. 21 is a top plan view showing another example of the link mechanism;
Fig. 22 is a chart showing an effect of another example of the structure; illustrating the relation between the angle of rotation of a crankshaft and the amount of movement of a slider; and
Fig. 23 is a conceptual diagram showing the conventional example.

### DESCRIPTION OF PREFERRED EMBODIMENTS

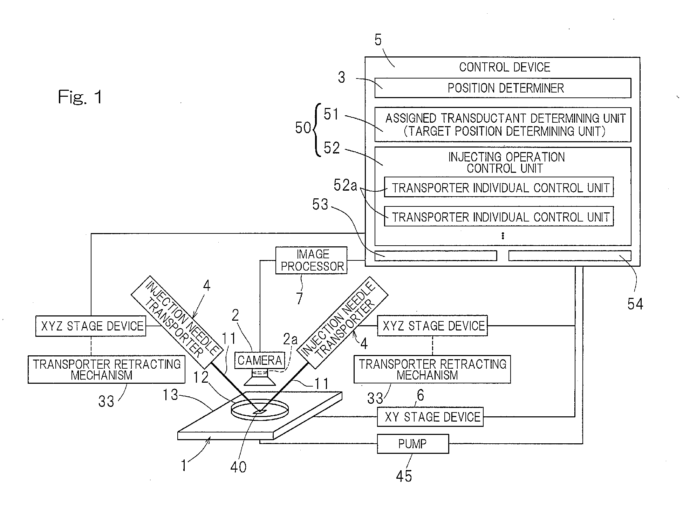

A first preferred embodiment of the present invention will be described in detail with particular reference to Figs. 1 to 9. Fig 1 illustrates a conceptual diagram of a microinjection apparatus according to the first embodiment of the present invention. The illustrated microinjection apparatus is an apparatus for introducing a transfecting material into a transductant, into which such transfecting material is to be introduced, by inserting a minute injection needle 11, having its interior filled with the transfecting material, into the transductant into which such transfecting material is to be introduced. The transductant is, for example, a biological cell. This cell may be a human cell or any other cell of a living creature such as, for example, any arbitrarily chosen animal or vegetable.

The illustrated microinjection apparatus includes a container position adjuster 1, a plurality of transporters 4, each of which is a manipulator employed for each of a plurality of injection needles 11, a transporter retracting mechanism 33 for selectively advancing or retracting the transporter 4 between a ready-to-inject position and a retracted position, an imaging device 2, and a control device 5 for controlling the operation of the apparatus as a whole. The control device 5 includes a position determiner 3, an assigned transductant determining unit51 and an injecting operation control unit 52.

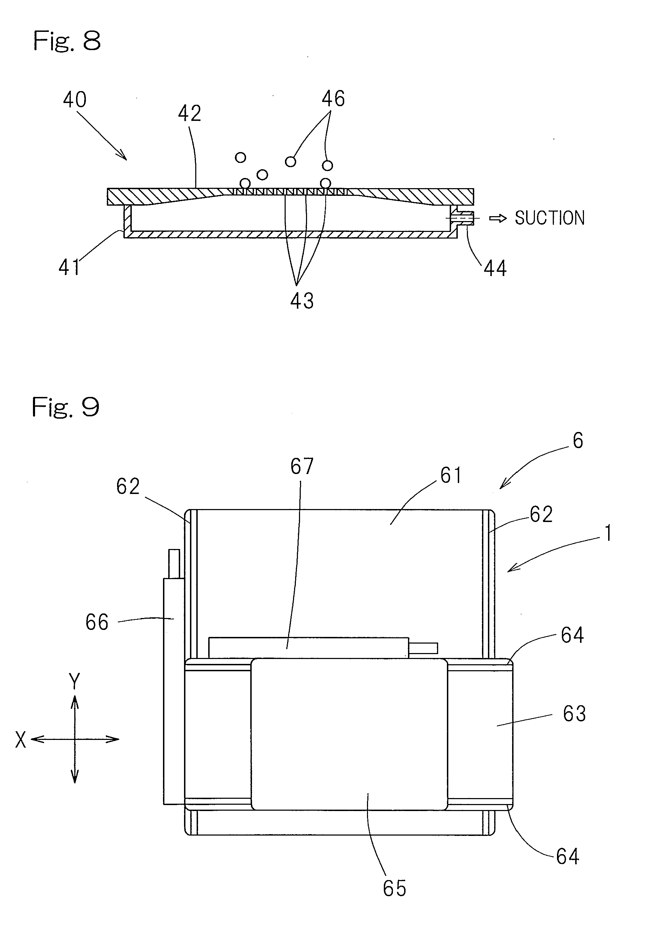

The container position adjuster 1 adjusts the position of a container 12 such as, for example, a petri dish or the like accommodating therein the transductant by moving a container support platform 13, on which the container is held in the form as placed, in horizontal biaxial directions perpendicular to each other and is comprised of an XY stage device 6. The XY stage device 6 includes, as shown in, for example, Fig. 9 in a top plan view, a machine bench 61, a lower movable stand 63 mounted on the machine bench 61 through a guide 62 for movement in a Y-axis direction, an upper movable stand 65 mounted on the lower movable stand 63 through a guide 64 for movement in an X-axis direction, and movable stand drive mechanisms 66 and 67 for selectively advancing or retracting the lower movable stand 63 and the upper movable stand 65, respectively. Each of the movable stand drive mechanisms 66 and 67 may be a linear motor or a rotary to linear motion translator of a kind comprised of, for example, a motor and a ball screw. The container support platform 13 referred to above is disposed on the upper movable stand 65, or the upper movable stand 65 itself forms the container support platform 13.

It is to be noted that the terms "X-axis", "Y-axis" and "Z-axis" are intended to represent respective axes relative to associated degrees of freedom in describing individual devices, not the respective axes in the rectangular coordinates defining the coordinate system common to the microinjection apparatus as a whole.

The imaging device 2 (best shown in Fig. 1) is employed only one in number and is disposed at a predetermined position above the container support platform 13 so as to look down upon the container 12 comprised of, for example, petri dish or the like. This imaging device 2 is in the form of, for example, a camera for taking an image such as, for example, an image as viewed in a plane illustrative of the interior of the container 12 on an enlarged scale, through a magnifying lens 2a, which image is processed by an image processor 7.

The position determiner 3 determines the position of the transductant in reference to the image captured by the imaging device 2 and is included as a part of, for example, the control device 5. This position determiner 3 determines the position of the transductant by checking a result of image processing by the image processor 7 with an appropriately preset setting standard. By way of example, the position determiner 3 determines the position of each transductant from the positional relation of the transductants appearing within the entire image.

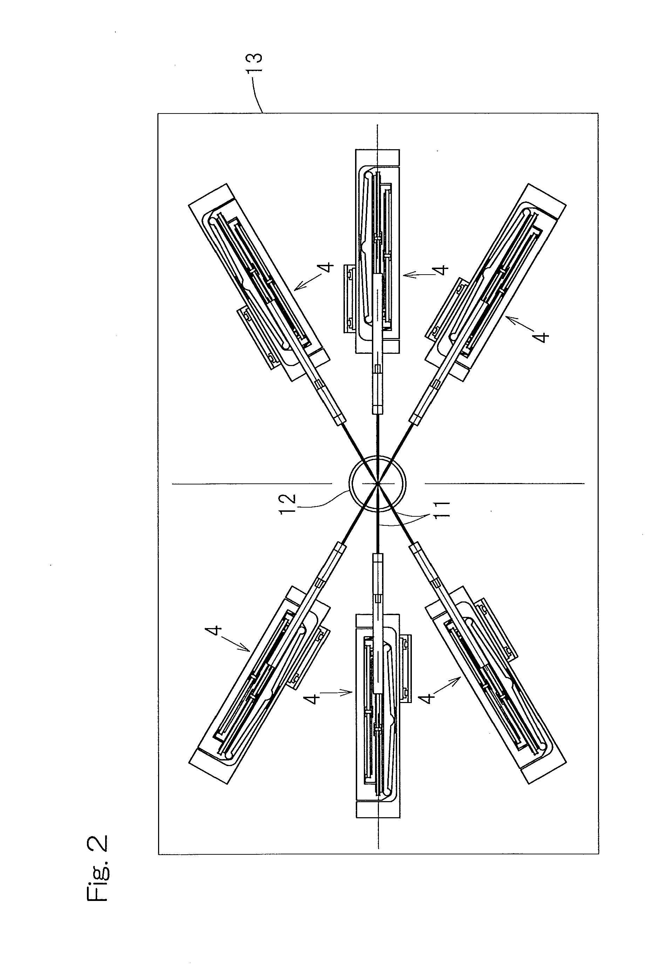

The transporter 4 is comprised of, for example, an XYZ stage device or the like for moving respective injection needles 11 and is employed in a plural number. Those plural transporters 4 are, as best shown in Fig. 2 in a top plan view, positioned above the container support platform 13 and arranged in a generally radial pattern so as to surround the container 12. In the illustrated instance, the transporter 4 is employed in a plural number (for example, three) on each side of the container 12 and the three transporters 4 on each side of the container 12 are spaced an equal distance from each other about the container 12, while the left and right transporters 4 are positioned in alignment with each other with the container 12 positioned at a point intermediate between one of the left transporters 4 and the right transporters 4 aligned with such one of the left transporters 4.

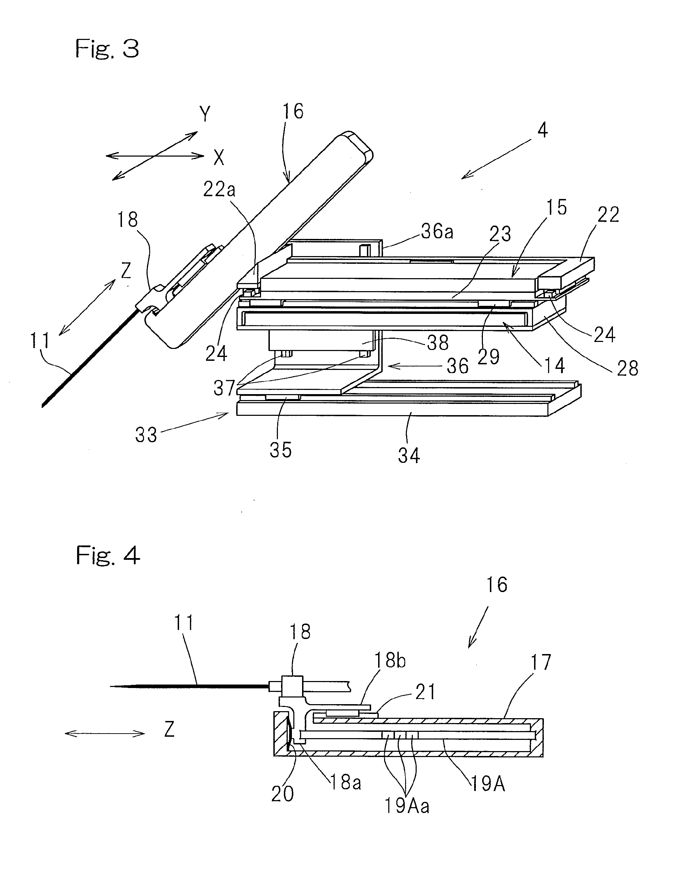

Fig. 3 illustrates a perspective view showing the entire construction of a retracting mechanism equipped transporter comprises of one transporter and a transporter retracting mechanism 33 for supporting the transporter 4. The transporter 4 has three degrees of freedom. A transfer mechanism responsible for one of the degrees of freedom is an X-axis transfer mechanism 14 for moving the injection needle 11 in one of two directions (X-axis and Y-axis directions) horizontally perpendicular to each other relative to the container support platform 13, which is an X-axis direction. A transfer mechanism responsible for another one of the degrees of freedom is a Y-axis transfer mechanism 15 for moving the injection needle 11 in another one of the two directions, that is, a Y-axis direction. Also, a transfer mechanism responsible for the rest of the degrees of freedom is a Z-axis transfer mechanism 16 for moving the injection needle 11 in a center axial direction parallel to the longitudinal axis of the injection needle 11 which is a direction inclined towards the interior of the container 12.

The Z-axis transfer mechanism 16 includes, as shown in Fig. 4 in a longitudinal sectional view, a stationary pedestal 17 of a hollow box-like configuration, a needle support member 18 provided at one end of the stationary pedestal 17 so as to protrude upwardly from the inside of the stationary pedestal 17 through an upper face opening thereof, and a piezoelectric elements laminate 19A arranged inside the stationary pedestal 17 and functioning as a drive source.

The needle support member 18 is of a structure that supports the injection needle 11 detachably, and the injection needle 11 can be easily replaced in the event of impairment of the injection needle 11 or replacement of the transfecting material. This needle support member 18 includes an upright piece 18a and a transverse piece 18b and is so shaped as to represent a configuration generally similar to the shape of a horizontally laid figure of T. This needle support member 18 detachably supports the injection needle 11 at an upper end of the upright piece 18a while the transverse piece 18b is movably supported on the stationary pedestal 17 through a guide mechanism 21 for movement in a direction parallel to the direction in which the injection needle 11 extends. A lower portion of the upright piece 18a of the needle support member 18 is supported by an inner wall face at one end of the stationary pedestal 17 through a spring member 20 which may be a leaf spring or the like.

The piezoelectric elements laminate 19A is of a structure in which a plurality of piezoelectric elements 19Aa are laminated one above the other in a direction conforming to the direction of displacement of those piezoelectric elements 19Aa so as to have a rod-like configuration. In the figure, only a part of the piezoelectric elements 19Aa are illustrated for the sake of brevity. The piezoelectric elements laminate 19A has one end connected with the upright piece 18a of the needle support member 18 and the other end supported by the other end of the stationary pedestal 17. The spring member 20 referred to previously applies a preload to the piezoelectric elements laminate 19A. Accordingly, in dependence upon displacement of the piezoelectric elements laminate 19A in a lengthwise direction, the needle support member 18 is selectively advanced or retracted in the direction parallel to the direction in which the injection needle 11 extends.

The Z-axis transfer mechanism 16 has a sensor (not shown) built therein for measuring a relative displacement between the stationary pedestal 17 and the needle support member 18. For the sensor, a strain sensor for detecting a strain occurring in the spring member 20 used to apply the preload to the piezoelectric elements laminate 19A, or one of sensors including, for example, an optical sensor, a magnetic sensor and an electric capacitance sensor for measuring a gap between the stationary pedestal 17 and the needle support member 18, can be employed

As best shown in Fig. 3, the Z-axis transfer mechanism 16 is fixedly mounted on an inclined mounting pedestal 22a at one end of an upper movable support body 22 so that the angle of injection of the injection needle 11 may attain a predetermined angle at which the injection needle 11 is oriented downwards. It is to be noted that this Z-axis transfer mechanism 16 may be so designed as to have an angle changing mechanism (not shown) for changing the angle of injection of the injection needle 11 as desired relative to the upper movable support body 22. The upper movable support body 22 is rendered to be of a rectangular frame shape having one side left open. This upper movable support body 22 is supported on a lower movable support body 23 of a generally flat plate configuration through left and right guide mechanisms 24 for horizontal movement in one direction (Y-axis direction). The Y-axis transfer mechanism 15 referred to previously is arranged on the lower movable support body 23. This Y-axis transfer mechanism 15 is mounted atop the lower movable support body 23. On the other hand, the lower movable support body 23 is supported on a transporter base 28 of a flat plate-like configuration, disposed therebelow, for movement in the X-axis direction through a guide mechanism 29. The X-axis transfer mechanism 14 is disposed below the transporter base 28.

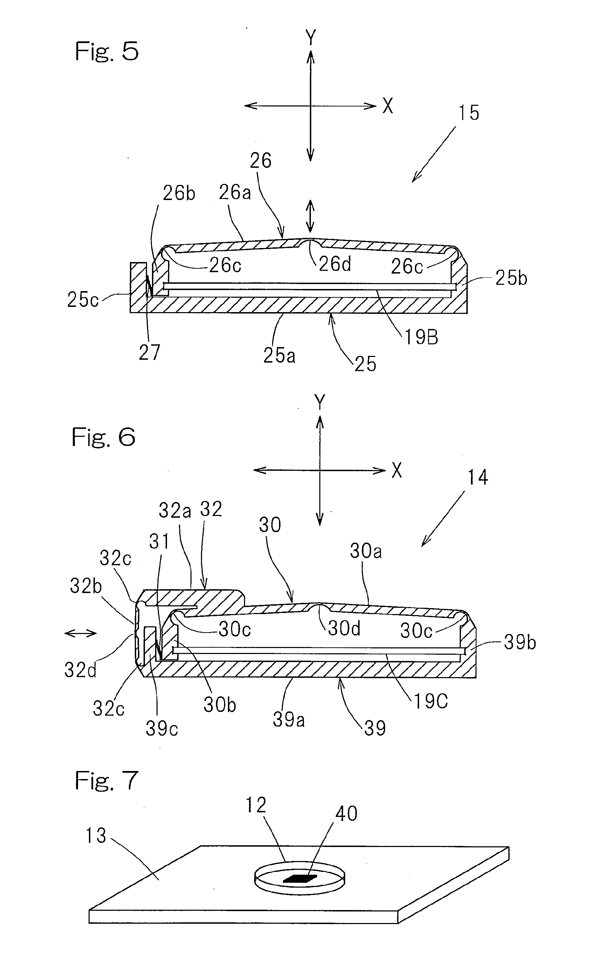

The Y-axis transfer mechanism 15 is shown in Fig. 5 in a horizontal sectional view. This Y-axis transfer mechanism 15 includes a stationary pedestal 25, a movable piece 26 and a piezoelectric elements laminate 19B functioning as a drive source. The stationary pedestal 25 includes a main frame portion 25a, extending in the X-axis direction, and a pair of side frame portions 25b and 25c both extending from opposite ends of the main frame portion 25a in a widthwise direction (Y-axis direction) and is of a U-shaped configuration in its horizontal sectional representation. The movable piece 26 is formed integrally with the stationary pedestal 25 and extends from the side frame portion 25b at one end of the stationary pedestal 25 to the other side frame portion 25c at the opposite end of the stationary pedestal 25. This movable piece 26 will form an amplifying mechanism for amplifying a displacement of the piezoelectric elements laminate 19B and is made of an elastic material such as, for example, metal or synthetic resin together with the stationary pedestal 25. The movable piece 26 is made up of a main frame parallel piece portion 26a extending from one of the side frame portion 25b of the stationary pedestal 25 in parallel relation with the main frame portion 25a, and a side frame parallel piece portion 26b expending from the opposite end of the main frame parallel piece portion 26a to the inside of the opposite side frame portion 25c of the stationary pedestal 25. The side frame parallel piece portion 26b extends parallel to the side frame portion 25c. A joint between the side frame portion 25b of the stationary pedestal 25 and the main frame parallel piece portion 26a of the movable piece 26 and a joint between the main parallel piece portion 26a and the side frame parallel piece portion 26b are rendered to be thin walled portions 26c. Also, a lengthwise intermediate portion of the main frame parallel piece portion 26a of the movable piece 26 is also rendered to be a thin walled portion 26d. Accordingly, the side frame parallel piece portion 26b of the movable piece 26 is pivotable about the axis of pivot that is defined by the thin walled portion 26c of a base end thereof. Also, the main frame parallel piece portion 26a of the movable piece 26 is bent at the thin walled portion 26d at a lengthwise intermediate portion thereof and, depending on increase or decrease of the angle of bending thereof, an intermediate portion is rendered to be selectively advanced or retracted in a direction (Y-axis direction) perpendicular to the lengthwise direction.

A spring member 27 such as, for example, a leaf spring for applying the preload to the piezoelectric elements laminate 19B is interposed between the side frame portion 25c at the other end of the stationary pedestal 25 and the side frame parallel piece portion 26b of the movable piece 26. The piezoelectric element laminate 19B is rendered to have a rod-like configuration having a laminated structure similar to that of the piezoelectric elements laminate 19A. The piezoelectric elements laminate 19B has one end connected with the side frame parallel piece portion 26b of the movable piece 26 and the other end supported by the side frame portion 25b of the stationary pedestal 25. Accordingly, displacement of the piezoelectric elements laminate 19B in a lengthwise direction thereof brings about a bending motion of the side frame parallel piece portion 26b of the movable piece 26, and such a bending displacement, after having been amplified, brings about a displacement of the main frame parallel piece portion 26a in the Y-axis direction. This displacement is transmitted to the support body 22 (shown in Fig. 3) for supporting the Z-axis transfer mechanism 16, causing the injection needle 11 to be movable in the Y-axis direction.

Even in the case of the Y-axis transfer mechanism 15, a sensor (not shown) is built therein for measuring a relative displacement between the stationary pedestal 25 and the movable piece 26. Even in the case of this sensor, a sensor similar to the sensor used in the case of the previously described Z-axis transfer mechanism 16 can be employed and, therefore, the details thereof are not reiterated for the sake of brevity.

The details of the X-axis transfer mechanism 14 is shown in Fig. 6 in a horizontal sectional view. The X-axis transfer mechanism 14 includes a stationary pedestal 39, a movable piece 30 and a piezoelectric elements laminate 19C functioning as a drive source. The stationary pedestal 39 includes a main frame portion 39a extending in the X-axis direction, and a pair of side frame portion 39b and 39c extending from opposite ends of the main frame portion 39a in a widthwise direction thereof and is rendered to have a horizontal section representing a U-shaped configuration. The movable piece 30 is formed integrally with the stationary pedestal 39 and extends from the side frame portion 39b at one end of the stationary pedestal 39 to the other side frame portion 39c at the opposite end of the stationary pedestal 39. This movable piece 30 will form a first amplifying mechanism for amplifying a displacement of the piezoelectric elements laminate 19C and is made of an elastic material such as, for example, metal or synthetic resin together with the stationary pedestal 39. The movable piece 30 is made up of a main frame parallel piece portion 30a extending from the side frame portion 39b at one end of the stationary pedestal 39 in parallel relation with the main frame portion 39a, and a side frame parallel piece portion 30b from the opposite end of the main frame parallel piece portion 30a to the opposite side frame portion 30b at the other end of the stationary pedestal 39 and is rendered to represent an L-shaped configuration in a horizontal section thereof. The side frame parallel piece portion 30b is positioned inside the side frame portion 39c and extends parallel to the side frame portion 39c. A spring member 31 such as, for example, a leaf spring for applying a preload to the piezoelectric elements laminate 19C is interposed between the side frame portion 39c of the stationary pedestal 39 and the side frame parallel piece portion 30b of the movable piece 30. A joint between the side frame portion 39b of the stationary pedestal 39 and the main frame parallel piece portion 30a of the movable piece 30 and a joint between the main parallel piece portion 30a and the side frame parallel piece portion 30b of the movable piece 30 are rendered to be thin walled portions 30c. Also, a lengthwise intermediate portion of the main frame parallel piece portion 30a of the movable piece 30 is also rendered to be a thin walled portion 30d. Accordingly, the side frame parallel piece portion 30b of the movable piece 30 is pivotable about the axis of pivot that is defined by the thin walled portion 30c of a base end thereof. Also, the main frame parallel piece portion 30a of the movable piece 30 is bendable and pivotable about the thin walled portion 30c at opposite ends thereof and is, at the same time, bent at the thin walled portion 30d at a lengthwise intermediate portion thereof so as to bend and pivot about the thin walled portion 30d.

A portion of the movable piece 30, which forms generally one of halves thereof and is situated adjacent the side frame parallel piece portion 30b and remote from the thin walled portion 30d at the lengthwise intermediate portion of the main frame parallel piece portion 30a of the movable piece 30, is formed integrally with a movable frame segment 32 of an inverted L-shaped configuration in a horizontal section, which segment 32 extends towards the side frame portion 39c of the stationary pedestal 39 and connected in the vicinity of a base end of the side frame portion 39c. This movable frame segment 32 will become a second amplifying mechanism for amplifying the displacement of the piezoelectric elements laminate 19C and is made up of a thick walled frame portion 32a substantially parallel to the main parallel frame piece portion 30a of the movable piece 30 and a thin walled frame portion 32b extending from the thick walled frame portion 32a at a location outside the side frame portion 39c of the stationary pedestal 39 in parallel relation with the side frame portion 39c. A joint between the thick walled frame portion 32a and the thin walled frame portion 32b of the movable frame segment 32 and a joint between the thin walled frame portion 32b and the vicinity of a base end of the side frame portion 39c of the stationary pedestal 39 are rendered to be respective further thin walled portions 32c. Also, a lengthwise intermediate portion of the thin walled frame portion 32b is also rendered to be a further thin walled portion 32d. Accordingly, the thin walled frame portion 32b of the movable frame segment 32 bends at the thin walled portion 32d at the lengthwise intermediate portion thereof and the thin walled portion 32c at the opposite ends thereof, thus, rendering the intermediate portion to be selectively advanced or retracted in a direction (X-axis direction) perpendicular to the lengthwise direction thereof.

The piezoelectric element laminate 19C is rendered to have a rod-like configuration having a laminated structure similar to that of the piezoelectric elements laminate 19A and has one end connected with the side frame parallel piece portion 30b of the movable piece 30 and the other end supported by the side frame portion 39b of the stationary pedestal 39. Accordingly, displacement of the piezoelectric elements laminate 19C in a lengthwise direction thereof brings about a pivotal motion of the side frame parallel piece portion 30b of the movable piece 30, and such a pivotal displacement, after having been amplified, brings about a displacement of the main frame parallel piece portion 30a in the Y-axis direction. This displacement is further amplified and then brings about a displacement of the rin walled portion 32b in the movable frame portion 32 in the X-axis direction. This displacement in the X-axis direction is transmitted to the lower movable support body 23 shown in Fig. 3, causing the injection needle 11 to be movable in the X-axis direction.

Even in the case of the X-axis transfer mechanism 14, a sensor (not shown) is built therein for measuring a relative displacement between the stationary pedestal 39 and the movable piece 32. Even in the case of this sensor, a sensor similar to the sensor used in the case of the previously described Z-axis transfer mechanism 16 can be employed and, therefore, the details thereof are not reiterated for the sake of brevity.

For the drive sources employed in the X-axis transfer mechanism 14, the Y-axis transfer mechanism 15 and the Z-axis transfer mechanism 16 of each of the transporters 4, other than the piezoelectric element laminates 19A, 19B and 19C, motors and ball screws, ultrasonic motors, linear motors, hydraulically operated actuators or pneumatically operated actuators may be employed. Also, in order to allow the tip of the injection needle 11 to be movable over the entire region of a predetermined field of view in the previously described image, the distance of movement of the injection needle 11 by the transporter 4 is preferably not smaller than 1 mm. The range of the predetermined field of view is arbitrarily chosen.

Referring to Fig. 3, the details of the transporter retracting mechanism 33 will be described. This transporter retracting mechanism 33 supports the transporter base 28 of the transporter 4 and is made up of a base table 34, an L-sectioned horizontally movable mount 36 and a vertically movable mount 38. The horizontally movable mount 36 is movable above the base table 34 in one horizontal direction (for example, the X-axis direction) through a guide mechanism 35. The vertically movable mount 38 is supported on an upright plate portion 36a of the horizontally movable mount 36 for movement in a vertical direction through a guide mechanism 37 and the transporter base 28 referred to previously is supported by this vertically movable mount 38. The horizontally movable mount 36 and the vertically movable mount 38 are driven by a drive source (not shown) such as, for example, a combination of a rotary motor and a ball screw, a linear motor or a hydraulically operated actuator. Accordingly, when the container 12 is to be detached upwardly of the container support platform 13 shown in Fig. 1, the horizontally movable mount 36 and the vertically movable mount 38 of the transporter retracting mechanism 33 have to be driven to allow the respective transporter 4 for the injection needle 11 to be retracted from an injection position so that a working space can be secured.

Referring to Fig. 1, the control device 5 will now be described in detail. The control device 5 controls the entire microinjection apparatus and includes a computer such as, for example, a microcomputer or a personal computer, a software program executed by such computer and electronic circuits. This control device 5 includes, other than the position determiner 3 referred to previously, the assigned transductant determining unit 51 and an injecting operation control unit 52, which units 51 and 52 form a controller 50 for the transporter 4.

The assigned transductant determining unit 51 determines as to which one of transductants injection should be made from the respective injection needle 11, on the basis of a preset rule or the like from the result of determination of the position of the respective transductant made by the position determiner 3, for example, from the positional relation of the respective transductant within the entire image and also determines injection target position as to what position of the determined transductant the injection needle 11 is to be inserted. With respect to the assignment of the transductant, it need not be specifically determined as "assigned" and the assignment of the transductant may be determined as a result of determination of a target position to which the tip of the respective injection needle 11 is moved. Also, the target position may be so determined that injection by the plural injection needles 11 can be made to the same transductant.

The injecting operation control unit 52 controls each of the transporters 4 so that the tip of the respective injection needle 11 can be moved to a target position determined by the assigned transductant determining unit 51. This injecting operation control unit 52 includes a transporter individual control unit 52a for performing a drive control of each of the transfer mechanisms 14 to 16 for the respective axes, which control unit 52a is employed for each of the transporters 4. The injecting operation control unit 52, if provided with an injection driver (not shown) for discharging the transfecting material into the injection needle 11, is provided with a controller (not shown) for the injection driver other than the above described control. Also, the injecting operation control unit 52 includes a retracting mechanism controller 53 for controlling each of the transporter retracting mechanisms 33 and a container position adjusting controller 54 for controlling each of the axes of the container position adjuster 1.

In the description that follows, the injecting operation performed by the microinjection apparatus of the structure hereinabove described will be described with particular reference to Fig. 1.

At the outset, by the drive of the container position adjuster 1 the container support platform 13 is moved horizontally to reposition the container 12 on the container support platform 13 to a predetermined position beneath the imaging device 2. Then, the imaging device 2 captures through the lens 2a, a plan view image of a portion of the container 12 that is locally enlarged. This image is processed by the image processor 7 and the position determiner 3 of the control device 5 determines from the image, the position of the cell, which is the transductant, within the container 12.

Thereafter, in dependence on a position information of each of the cells obtained from the position determiner 3, the cell for which the respective injection needle 11 is assigned to inject and the target position at which the tip of the injection needle 11 is to be inserted are determined by the assigned transductant determining unit 51. In response to the determination so made, each of the transporters 4 supporting the respective injection needles 11 is controlled by the associated transporter individual control unit 52a of the injecting operation control unit 52. In such case, the injecting operation control unit 52 has to be operated so that the injection needles 11 in the transporters 4 will not interfere with each other.

In this way, for example, the following injecting operation by means of each of the injection needles 11 is performed. That is to say, as an injecting operation, positioning of each of the injection needles 11 in a horizontal direction and an injecting direction is performed, the injection needle 11 is inserted into the cell determined by the movement of the injection needle 11 in the injecting direction, and the transfecting material filled within the injection needle 11 is introduced into the cell. This introduction may be accomplished by either discharging the transfecting material by the drive from the injection needle 11 or introducing as a result of penetration by the effect of osmotic pressure. The whole of a series of those injecting operations performed by the injection needles 11 are controlled by the control device 5. During the movement of the injection needle 11 in the injecting direction, vibration may be effected as a reciprocal movement in order that a smooth insertion into the cell can be accomplished. Also, the frequency of vibration can be altered depending on the kind of the cell, or depending whether a cell membrane is pierced or whether a nuclear membrane is pierced. The transfecting material is a gene regulatory factor such as, for example, DNA or protein.

After completion of the process of injection into the cell within the image, the container support platform 13 is horizontally moved a predetermined quantity by the drive of the container position adjuster 1 so that a cell position at a different position proximate to the previous image capturing position within the container 12 can be recognized by the position determiner 3 from the image captured by the imaging device 2, followed by repetition of the above described cycle of injecting operation.

As hereinabove described, with the microinjection apparatus of the structure hereinbefore described, the transfecting material such as, for example, the gene regulatory factor is introduced into each of the cells by adjusting the position of the container 12 accommodating, for example, a cell as the transductant, by means of the container position adjuster 1, capturing a plan view image of the inside of the container 12, then held at the adjusted position, by means of the imaging device 2, determining the cell position from such image by means of the position determiner 3 to determine the cell for which the injection needle 11 is assigned, and moving the plurality of the transporters 4 together with the respective injection needles 11 to allow the respective injection needle 11 to be inserted into each of the cells. For this reason, the injecting operation to the plurality of the cells can be paralleled all at one time and, therefore, the efficiency of the injecting process can be considerably increased.

Also, the microinjection apparatus of the structure as hereinbefore described can be so configured to give rise to a high injecting process efficiency merely by adding a plurality of transporters 4, corresponding to a plurality of injection needles 11, in the conventional apparatus shown in Fig. 23.

In addition, since in the embodiment described hereinbefore, the plurality of the transporters 4 for individually moving the associated injection needles 11 are arranged in a radial pattern around the container 12 for accommodating the transductant, the plurality of the transporters 4 can be arranged in a limited space available around the container 12 and, therefore, a correspondingly increased number of the injection needles 11 can be used to thereby further increase the efficiency of the injecting process. It is to be noted that such arrangement of the transporters 4 as hereinabove described is realized because the individual transporter 4 can perform respective transport operations for the associated injection needle 11 separately.

Moreover, although in the embodiment described hereinbefore, the transporter 4 for the injection needle 11 have been described as having the transfer mechanisms having the three degrees of freedom, that is, the X-axis transfer mechanism 14, the Y-axis transfer mechanism 15 and the Z-axis transfer mechanism 16, positioning of the tip of the injection needle 11 by means of the single transporter can be accomplished if the transporter has a transfer mechanism having at least two degrees of freedom.

Yet, in the embodiment hereinbefore described, since the use is made of the piezoelectric elements laminates 19A, 19B and 19C as respective drive sources for the X-axis transfer mechanism 14, the Y-axis transfer mechanism 15 and the Z-axis transfer mechanism 16 of the transporter 4, the transporter 4 can be structured compact in size and therefore the efficiency of the injecting process can further be increased. The piezoelectric elements laminates 19A,19B and 19C have a high conversion efficiency of converting electric energies into mechanical energies and, hence, if a voltage applied thereto is changed, the displacement generated can be relatively easily varied, bringing about a meritorious feature in controllability.

In the meantime, the cell, which is used as the transductant, includes, for example, an attachment cell, attached to a culture media and a suspended cell suspended within a culture fluid. In the case of the attachment cell, the injecting operation can be performed in the manner as hereinbefore described. Figs. 7 and 8 illustrate an example of the structure of a cell adsorbing substrate unit that is used in the case where the transductant is the suspended cell. The illustrated cell adsorbing substrate unit 40 is of a structure in which the opening of an open-topped box 41 is closed with a cell adsorbing substrate 42 having a plurality of micropores 43 and a portion of the box 41 is provided with a suction port 44 for sucking a culture fluid from the inside of the box 41. Depending on the size of the cell to be subjected to injection, the cell adsorbing substrates 42 is prepared in a plural number having the micropores of different pore sizes. In the microinjection apparatus shown in Fig. 1, a pump 45, which is used when the cell adsorbing substrate unit 40 of the structure described above is used, is provided in anticipation of the use thereof with the cell adsorbing substrate unit 40. Hence, by sucking the culture fluid through the suction port 44 of the cell adsorbing substrate unit 40 by means of the pump 45, the suspended cells 46 can be fixed in position in the micropores 43 in the cell adsorbing substrate 42. A controller (not shown) for controlling the pump 45 is also employed in the control device 5.

Also, the injection method, which does not make use of the previously described cell adsorbing substrate unit 40, but is applicable where the transductant is the suspended cell, will now be described. At least two or more transporters, each similar to the transporter 4 for the injection needle 11, are combined, and the injection process is performed with the injection needle 11 by causing the injection needle 11 to be supported by one of the transporters, whereas the other of the transporters is used to hold a tube for fixing the cells, and then by sucking and fixing the suspended cells through the tube. In this case, it is possible to move the cells to an arbitrarily desired position within the container 12, by means of a manipulation on the side of the tube for fixing the cells. In other words, the cells can be classified by means of the manipulation of the transporter 4 then holding the tube for fixing the cells.

According to the microinjection apparatus of the structure described hereinbefore, where the transfecting materials filled respectively within the plurality of the injection needles 11 are the same transfecting material for all of those injection needles 11, the speed of the injecting operation can be increased to a value n-times that according to the conventional case, wherein n represents the number of the injection needles 11.

Also, where the transfecting materials filled respectively within the plurality of the injection needles 11 are different from each other, by controlling the transporter 4 so as to perform an operation of successively inserting the plural injection needles 11 to carry out the injection into the same cells, it is possible to inject the plural transfecting materials, that are different from each other, into single cell. The injecting operation control unit 52 may be so configured as to perform such a control that the plural transporter 4 can be successively operated in the manner described above.

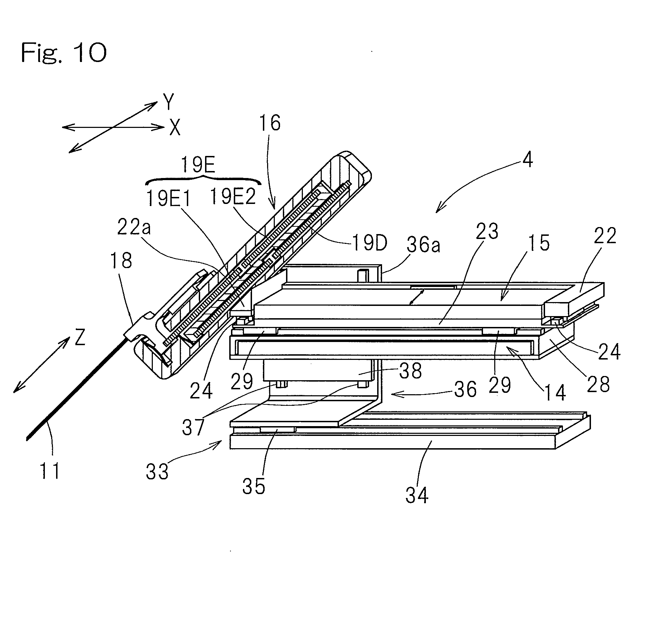

A second preferred embodiment of the present invention will be described in detail with particular reference to Figs. 10 to 22. This second embodiment is similar to the previously described first embodiment of the present invention, but differs therefrom in that the transporter 4 has at least two degrees of freedom and includes a transfer mechanism for each of the degrees of freedom and in that of those transfer mechanisms, the at least one transfer mechanism has as a drive source a plurality of piezoelectric elements laminates, which are each comprised of a plurality of piezoelectric elements and which are capable of undergoing expansion and contraction in a direction conforming to the direction in which the piezoelectric elements are laminated, in a fashion arranged one above the other in parallel relation to each other. It is to be noted that component parts employed in the second embodiment, which are similar to those employed in the first embodiment, are designated by like reference numerals used in connection with the previously described first embodiment and, therefore, the details thereof are not reiterated for the sake of brevity. It is also to be noted that in the description of the second embodiment, reference to Figs. 1, 2 and 7 to 9, which are employed in the description of the first embodiment will be employed as it stands.

Fig. 10 illustrates a perspective view showing the entire construction of the retracting mechanism equipped transporter comprised of one transporter 4 and a transporter retracting mechanism 39 for supporting such transporter 4. Fig. 10 corresponds to Fig. 3, reference to which has already been made in describing the first embodiment.

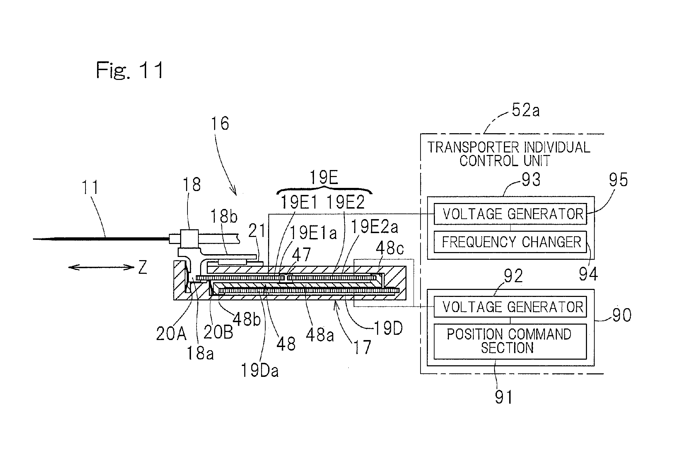

Fig. 11 illustrates one example of the structure of the Z-axis transfer mechanism 16. Fig. 11 corresponds to Fig. 4 which has been referred to in describing the first embodiment, and, in place of the piezoelectric elements laminate 19A shown in and described with reference to Fig. 4, a piezoelectric elements laminate 19D and piezoelectric elements laminates 19E1 and 19E2 arranged parallel to the piezoelectric element laminate 19D are employed. A spring member 20A employed in this structural example shown in Fig. 11 corresponds to the spring member 20 shown in and described with reference to Fig. 4.

Each of the piezoelectric elements laminates 19D, 19E1 and 19E2 is a laminated piezoelectric element of a rod-like configuration formed by laminating a plurality of piezoelectric elements 19Da, 19E1a or 19E2a in a direction conforming to the direction of displacement thereof as is the case with the previously described piezoelectric elements laminate 19A. Of those piezoelectric elements laminates 19D, 19E1 and 19E2, the two piezoelectric elements laminates 19E1 and 19E2 are rendered to be one set of a piezoelectric elements laminate 19E comprised of a coupled body in which those two piezoelectric elements laminates 19E1 and 19E2 are arranged in line with each other and connected in series with each other by means of a fastening member 47. The piezoelectric elements laminate 19E and the remaining piezoelectric elements laminate 19D are arranged parallel to each other and positioned one above the other in a direction conforming to the direction of lamination of the constituent piezoelectric elements and those two set of the piezoelectric elements laminates 19D and 19E are connected in series with each other through a fastening member 48. The fastening member 48 is of a generally Z-shaped configuration including a lengthwise segment 48a, arranged between the upper and lower piezoelectric elements laminates 19D and 19E in parallel relation therewith, and projecting segments 48b and 48c situated at both ends of the lengthwise segment 48a and projecting in up and down directions so as to assume an inverted relation to each other. The set of the piezoelectric elements laminate 19D has one end coupled to the projecting segment 48b of the fastening member 48 adjacent one end portion of the stationary pedestal 17 and the other end supported by the other end portion of the stationary pedestal 17. A spring member 20B such as, for example, a leaf spring for applying a preload to the piezoelectric elements laminate 19D is interposed between the projecting segment 48b of the fastening member 48 and one end portion of the stationary pedestal 17. One end portion of the piezoelectric elements laminate 19E, that is, one end portion of one 19E1 of the piezoelectric elements laminates (constituting the piezoelectric elements laminate 19E) that is remote from a joint defined by the fastening member 47, is connected with the upright piece 18a of the needle support member 18. Also, the opposite end portion of the piezoelectric elements laminate 19E, that is, the opposite end portion of the other 19E2 of the piezoelectric elements laminates (constituting the piezoelectric elements laminate 19E) that is remote from the joint defined by the fastening member 47, is connected with the projecting segment 48c of the fastening member 48, which is oriented towards the other end portion of the stationary pedestal 17. A preload is applied to the piezoelectric elements laminate 19E through the spring member 20A such as, for example, a leaf spring that is interposed between the upright piece 18a of the needle support member 18 and the other end portion of the stationary pedestal 17. Accordingly, displacement of the two sets of the piezoelectric elements laminates 19D and 19E in the direction conforming to the direction of lamination makes it possible for the needle support member 18 to selectively advance or retract in a direction parallel to the direction of projection of the injection needle 11.

Of the piezoelectric element laminates 19D and 19E, the piezoelectric elements laminate 19D and one 19E2 of the piezoelectric element laminates forming the piezoelectric elements laminate 19E are used as a drive source for positioning the needle support member 18, that is, for positioning the injection needle 11. In other words, those piezoelectric elements laminates 19D and 19E2 are displaced in response to a positioning signal, which is a voltage applied from a Z-axis (direction of insertion) positioning control unit 90.

Of the piezoelectric elements laminates 19D and 19E, the piezoelectric element laminate 19E1 in the piezoelectric element laminate 19E, which is connected directly with the needle support member 18, is used as a drive source for vibrating the needle support member 18 in the direction of insertion of the injection needle 11. Displacement of the piezoelectric element laminate 19E1 that is used to apply the vibration changes repeatedly in response to a vibration drive signal, which is a voltage applied from a vibration driver 93.

The Z-axis positioning control unit 90 is so constructed that a position command can be applied from a position command section 91 to a voltage generator 92 and, based on such position command, respective voltages can be applied from the voltage generator 92 to the piezoelectric element laminates 19D and 19E2. The Z-axis positioning control unit 90 is provided in the transporter individual control unit 52a for the Z-axis, which is included in the injecting operation control unit 52 best shown in Fig. 1. Also, based on the position information obtained by the position determiner 3 best shown in Fig. 1, the position command section 91 thereof makes the target position, determined by the assigned transductant determining unit 51, to be the position command referred to above. This position command section 91 may be provided as a part of the assigned transductant determining unit 51.

The vibration driver 93 applies an alternating voltage of a predetermined frequency as the vibration drive signal from the voltage generator 95 to the piezoelectric element laminate 19E1. The frequency of such alternating voltage, that is, the frequency of vibrations applied to the needle support member 18 is rendered to be altered in response to a command fed from a frequency changer 94 to the voltage generator 95.

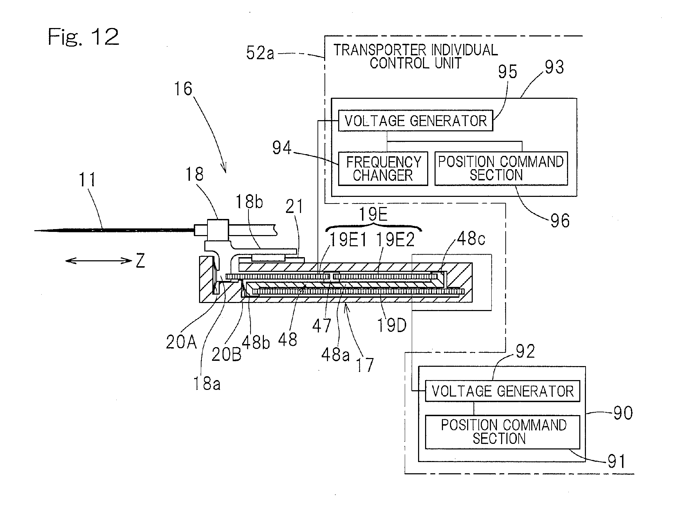

Fig. 12 illustrates another example of the structure of the Z-axis transfer mechanism 16. This example of the structure is such that in the example of the structure shown in Fig. 11, a position command section 96 is added to the vibration driver 93, which position command section 96 applies a position command to the voltage generator 95 based on the position information obtained by the position determiner 3 shown in Fig. 1. Accordingly, the vibration driver 93 applies a voltage, in which a positioning signal necessary to reposition the needle support member 18 and the vibration drive signal are superimposed with each other, to the piezoelectric elements laminate 19E1. The position command section 96 may be provided as a part of the assigned transductant determining unit 51 shown in and described with reference to Fig. 1. Other structural features are similar to those of the example of the structure shown in and described with reference to Fig. 11.

The Z-axis transfer mechanism 16 has a sensor (not shown) built therein for determining the relative displacement between the stationary pedestal 17 and the needle support member 18. For this sensor, a sensor similar to what has been described hereinbefore can be employed.

As best shown in Fig. 10, the Z-axis transfer mechanism 16 is fixedly mounted on an inclined mounting pedestal 22a at one end of an upper movable support body 22 so that the angle of injection of the injection needle 11 may attain a predetermined angle at which the injection needle 11 is oriented downwards. It is to be noted that this Z-axis transfer mechanism 16 may be so designed as to have an angle changing mechanism (not shown) for changing the angle of injection of the injection needle 11 as desired relative to the upper movable support body 22. The upper movable support body 22 is rendered to be of a rectangular frame shape having one side left open. This upper movable support body 22 is supported on a lower movable support body 23 of a generally flat plate configuration through left and right guide mechanisms 24 for horizontal movement in one direction (Y-axis direction). The Y-axis transfer mechanism 15 referred to previously is arranged on the lower movable support body 23. This Y-axis transfer mechanism 15 is mounted atop the lower movable support body 23. On the other hand, the lower movable support body 23 is supported on a transporter base 28 of a flat plate-like configuration, disposed therebelow, for movement in the X-axis direction through a guide mechanism 29. The X-axis transfer mechanism 14 is disposed below the transporter base 28.

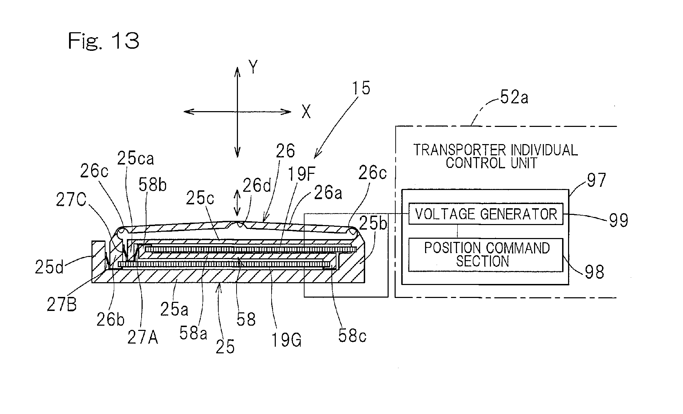

The Y-axis transfer mechanism 15 is shown in Fig. 13 in a horizontal sectional view. This Y-axis transfer mechanism 15 includes a stationary pedestal 25, a movable piece 26 and two sets of piezoelectric elements laminates 19F and 19G each functioning as a drive source. The stationary pedestal 25 is of a hollow box-like shape and includes a main frame portion 25a, extending in the X-axis direction, a pair of side frame portions 25b and 25d both extending from opposite ends of the main frame portion 25a in a widthwise direction (Y-axis direction), and an auxiliary frame portion 25c of an L-shaped horizontal section extending from a tip end of a side frame portion 25b in the X-axis direction in parallel to the main frame portion 25a. The movable piece 26 is of an L-shaped horizontal section and extends from a tip end of the side frame portion 25b at one end of the stationary pedestal 25 towards the side frame portion 25d at the opposite end thereof. This movable piece 26 will form an amplifying mechanism for amplifying a displacement of each of the piezoelectric elements laminates 19F and 19G and is made of an elastic material such as, for example, metal or synthetic resin together with the stationary pedestal 25. The movable piece 26 is made up of a main frame parallel piece portion 26a extending from one of the side frame portion 25b of the stationary pedestal 25 in substantially parallel relation with the main frame portion 25a, and a side frame parallel piece portion 26b extending from the other end of the main frame piece portion 26a to the inside of the side frame portion 25d at the other end of the stationary pedestal 25 in parallel relation with the side frame portion 25d. A joint between the side frame portion 25b of the stationary pedestal 25 and the main frame parallel piece portion 26a of the movable piece 26 and a joint between the main parallel piece portion 26a and the side frame parallel piece portion 26b are rendered to be thin walled portions 26c. Also, a lengthwise intermediate portion of the main frame parallel piece portion 26a of the movable piece 26 is also rendered to be a thin walled portion 26d. Accordingly, the side frame parallel piece portion 26b of the movable piece 26 is rendered to be so pivotable about the axis of pivot as to bend, which axis is defined by the thin walled portion 26c of a base end thereof. Also, the main frame parallel piece portion 26a of the movable piece 26 is bent at the thin walled portion 26d at a lengthwise intermediate portion thereof and, depending on increase or decrease of the angle of bending thereof, an intermediate portion is rendered to be selectively advanced or retracted in a direction (Y-axis direction) perpendicular to the lengthwise direction.

The two sets of the piezoelectric elements laminates 19F and 19G are both laminated type piezoelectric elements and are juxtaposed forwards and rearwards in parallel relation to each other so as to assume a parallel relation with the main frame portion 25a of the stationary pedestal 25 along the direction of lamination thereof. Those two sets of the piezoelectric elements laminates 19F and 19G are connected in series with each other by means of a fastening member 58. The fastening member 58 is is of a generally Z-shaped configuration including a lengthwise segment 58a disposed between the piezoelectric elements laminates 19F and 19G, positioned forwards and rearwards, respectively, in parallel relation with the piezoelectric elements laminates 19F and 19G, and projecting segments 58b and 58c situated at both ends of the lengthwise segment 58a and projecting in a forward and rearward direction so as to assume an inverted relation to each other. The set of the piezoelectric elements laminate 19F is supported at one end by the side frame portion 25b of the stationary pedestal 25 and is connected at the opposite end with the projecting segment 58b of the fastening member 58 which is oriented towards the stationary pedestal side frame portion 25d. Also, the remaining set of the piezoelectric elements laminate 19G is connected at one end with the projecting segment 58c of the fastening member 58, which is oriented towards the stationary pedestal side frame portion 25b, and at the opposite end with the side frame parallel piece portion 26b of the movable piece 26. A spring member 27A such as, for example, a leaf spring for applying a preload to the piezoelectric elements laminate 19F is interposed between a side segment 25ca at a tip end of the auxiliary frame portion 25c, which extends in a widthwise direction, and the projecting segment 58b of the fastening member 58. A spring member 27B such as, for example, a leaf spring for applying a preload to the piezoelectric elements laminate 19G, is interposed between the side frame portion 25b of the stationary pedestal 25 and the side frame parallel piece portion 26b of the movable piece 26. Also, a spring member 27C such as, for example, a leaf spring for applying a preload to the side frame parallel piece portion 26b of the movable piece 26 is interposed between the side segment 25ca at the tip end of the auxiliary frame portion 25c, which extends in a widthwise direction, and the side frame parallel piece portion 26b of the movable piece 26. Accordingly, displacement of the two sets of the piezoelectric elements laminates 19F and 19G resulting from expansion or contraction in the direction of lamination brings about a pivotal movement of the side frame parallel piece portion 26b of the movable piece 26 and such pivotal displacement, after having been amplified, brings about a displacement of the main frame parallel piece portion 26a in the Y-axis direction. This displacement is transmitted to the upper movable support body 22 (best shown in Fig. 10) supporting the Z-axis transfer mechanism 16, thus making it possible for the injection needle 11 to move in the Y-axis direction.

The two sets of the piezoelectric elements laminates 19F and 19G undergo a displacement in response to the positioning signal, which is a voltage applied from a Y-axis position control unit 97. The Y-axis position control unit 97 is so constructed that a position command can be applied from a position command section 98 to a voltage generator 99 and, based on such position command, respective voltages can be applied from the voltage generator 99 to the piezoelectric element laminates 19F and 19G The Y-axis position control unit 97 is provided in the transporter individual control unit 52a for the Y-axis in the injecting operation control unit 52 best shown in Fig. 1. Also, based on the position information obtained by the position determiner 3 best shown in Fig. 1, the position command section 98 makes the target position, determined by the assigned transductant determining unit 51, to be the position command referred to above. This position command section 98 may be provided as a part of the assigned transductant determining unit 51.

Even the Y-axis transfer mechanism 15 has a sensor (not shown) built therein for determining the relative displacement between the stationary pedestal 25 and the movable piece 26. For this sensor, a sensor similar to what has been described hereinbefore can be employed.

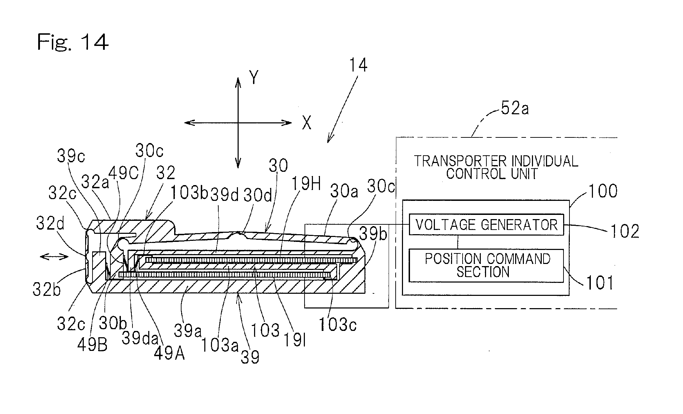

Fig. 14 illustrates the X-axis transfer mechanism 14 in a horizontal sectional view. The X-axis transfer mechanism 14 is of a hollow box-like shape and includes a stationary pedestal 39, a movable piece 30 and two sets of piezoelectric elements laminates 19H and 19I functioning as a drive source. The stationary pedestal 39 includes a main frame portion 39a extending in the X-axis direction, and a pair of side frame portion 39b and 39c extending from opposite ends of the main frame portion 39a in a widthwise direction (Y-axis direction), and an auxiliary frame portion 39d of an L-shaped horizontal section extending from a tip end of a side frame portion 39b in the X-axis direction in parallel to the main frame portion 39a. The movable piece 30 is rendered to be of an L-shaped horizontal section and extends from a tip end of the side frame portion 39b at one end of the stationary pedestal 39 towards the side frame portion 39c at the opposite end thereof. This movable piece 30 will form a first amplifying mechanism for amplifying a displacement of each of the piezoelectric elements laminates 19H and 19I and is made of an elastic material such as, for example, metal or synthetic resin integrally with the stationary pedestal 39. The movable piece 30 is made up of a main frame parallel piece portion 30a extending from one of the side frame portion 39b of the stationary pedestal 39 in substantially parallel relation with the main frame portion 39a, and a side frame parallel piece portion 30b extending from the other end of the main frame piece portion 30a to the inside of the side frame portion 39c at the other end of the stationary pedestal 39 in parallel relation with the side frame portion 39c. A joint between the side frame portion 39b of the stationary pedestal 39 and the main frame parallel piece portion 30a of the movable piece 30 and a joint between the main parallel piece portion 30a and the side frame parallel piece portion 30b of the movable piece 30 are rendered to be thin walled portions 30c. Also, a lengthwise intermediate portion of the main frame parallel piece portion 30a of the movable piece 30 is also rendered to be a thin walled portion 30d. Accordingly, the side frame parallel piece portion 30b of the movable piece 30 is rendered to be so bendable about the axis of pivot as to pivot in a direction (Y-axis direction) perpendicular to the lengthwise direction, which axis is defined by the thin walled portion 30c of a base end thereof. The structure so far described above is similar to that of the Y-axis transfer mechanism 15 best shown in and described with reference to Fig. 13.

Also, a portion of the movable piece 30, which forms generally one of halves thereof and is situated adjacent the side frame parallel piece portion 39b and remote from the thin walled portion 30d at the lengthwise intermediate portion of the main frame parallel piece portion 30a of the movable piece 30, is formed integrally with a movable frame segment 32 of an inverted L-shaped configuration in a horizontal section, which segment 32 extends towards the side frame portion 39d of the stationary pedestal 39 and connected in the vicinity of a base end of the side frame portion 39c. This movable frame segment 32 will become a second amplifying mechanism for amplifying the displacement of the piezoelectric elements laminates 19H and 19I and is made up of a thick walled frame portion 32a substantially parallel to the main parallel frame piece portion 30a of the movable piece 30 and a thin walled frame portion 32b extending from the thick walled frame portion 32a at a location outside the side frame portion 39c of the stationary pedestal 39 in parallel relation with the side frame portion 39c. A joint between the thick walled frame portion 32a and the thin walled frame portion 32b of the movable frame segment 32 and a joint between the thin walled frame portion 32b and the vicinity of the base end of the side frame portion 39c of the stationary pedestal 39 are rendered to be respective further thin walled portions 32c each having a thickness smaller than that of the thin walled frame portion 32b. Also, a lengthwise intermediate portion of the thin walled frame portion 32b is also rendered to be a further thin walled portion 32d. Accordingly, the thin walled frame portion 32b of the movable frame segment 32 bends at the thin walled portion 32d with the lengthwise intermediate portion thereof consequently capable of advancing or retracting in a direction (X-axis direction) perpendicular to the lengthwise direction.

As is the case with the Y-axis transfer mechanism 15 shown in and described with reference to Fig. 13, the two sets of the piezoelectric elements laminates 19H and 19I are both laminated type piezoelectric elements and are juxtaposed forwards and rearwards in parallel relation to each other so as to assume a parallel relation with the main frame portion 39a of the stationary pedestal 39 along the direction of lamination thereof. Those two sets of the piezoelectric elements laminates 19H and 19I are connected in series with each other by means of a fastening member 103. The fastening member 103 is of a generally Z-shaped configuration including a lengthwise segment 103a disposed between the piezoelectric elements laminates 19H and 19I, positioned forwards and rearwards, respectively, in parallel relation with the piezoelectric elements laminates 19H and 19I, and projecting segments 103b and 103c situated at both ends of the lengthwise segment 103a and projecting in a forward and rearward direction so as to assume an inverted relation to each other. The set of the piezoelectric elements laminate 19H is supported at one end by the side frame portion 39b of the stationary pedestal 39 and is connected at the opposite end with the projecting segment 103b of the fastening member 103 which is oriented towards the stationary pedestal side frame portion 39c. Also, the remaining set of the piezoelectric elements laminate 19I is connected at one end with the projecting segment 103c of the fastening member 103, which is oriented towards the stationary pedestal side frame portion 39c, and at the opposite end with the side frame parallel piece portion 30b of the movable piece 30. A spring member 49A such as, for example, a leaf spring for applying a preload to the piezoelectric elements laminate 19H is interposed between a side segment 39da at a tip end of the auxiliary frame portion 39d of the stationary pedestal 39, which extends in a widthwise direction, and the projecting segment 103b of the fastening member 103. A spring member 49B such as, for example, a leaf spring for applying a preload to the piezoelectric elements laminate 19I, is interposed between the side frame portion 39c of the stationary pedestal 39 and the side frame parallel piece portion 30b of the movable piece 30. Also, a spring member 49C such as, for example, a leaf spring for applying a preload to the side frame parallel piece portion 30b of the movable piece 30 is interposed between the side segment 39da at the tip end of the auxiliary frame portion 39d of the stationary pedestal 39, which extends in a widthwise direction, and the side frame parallel piece portion 30b of the movable piece 30. Accordingly, displacement of the two sets of the piezoelectric elements laminates 19H and 19I resulting from expansion or contraction in the direction of lamination brings about a pivotal movement of the side frame parallel piece portion 30b of the movable piece 30 and such pivotal displacement, after having been amplified, brings about a displacement of the main frame parallel piece portion 30a in the Y-axis direction. This displacement is further magnified and brings about a displacement of the thin walled portion 32b of the movable frame segment 32 in the X-axis direction. This displacement in the X-axis direction is transmitted to the lower movable support body 23 best shown in Fig. 10, thus making it possible for the injection needle 11 to move in the X-axis direction.

The two sets of the piezoelectric elements laminates 19H and 19I undergo a displacement in response to the positioning signal, which is a voltage applied from a X-axis position control unit 100. The X-axis position control unit 100 is so constructed that a position command can be applied from a position command section 101 to a voltage generator 102 and, based on such position command, respective voltages can be applied from the voltage generator 102 to the piezoelectric element laminates 19H and 19I. The X-axis position control unit 100 is provided in the transporter individual control unit 52a for the X-axis in the injecting operation control unit 52 best shown in Fig. 1. Also, based on the position information obtained by the position determiner 3 best shown in Fig. 1, the position command section 101 makes the target position, determined by the assigned transductant determining unit 51, to be the position command referred to above. This position command section 101 may be provided as a part of the assigned transductant determining unit 51.

Even the X-axis transfer mechanism 14 has a sensor (not shown) built therein for determining the relative displacement between the stationary pedestal 39 and the movable piece 30. For this sensor, a sensor similar to what has been described hereinbefore can be employed.

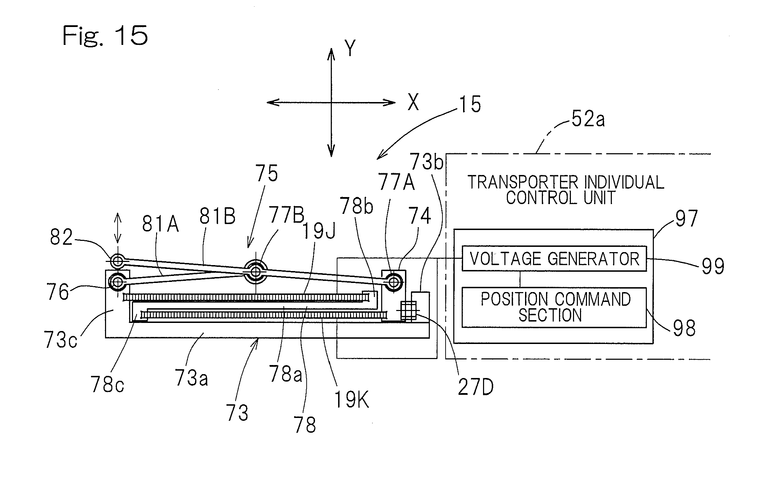

Fig. 15 illustrates another example of the structure of the Y-axis transfer mechanism 15. As is the case with the example of the structure shown in and described with reference to Fig. 13, even this example of the structure includes a stationary pedestal 73 and two sets of piezoelectric elements laminates 19J and 19K functioning as a drive source. The stationary pedestal 73 includes a main frame portion 73a, extending in the X-axis direction, and a pair of side frame portions 73b and 73c both extending from opposite ends of the main frame portion 73a in a widthwise direction (Y-axis direction), A expansion and contraction direction movable body 74 movable in a direction of expansion and contraction is supported by the side frame portion 73b at the one end of the stationary pedestal 73 for movement in the X-axis direction so as to confront the side frame portion 73c at other end of the stationary pedestal 73.

Two sets of piezoelectric elements laminates 19J and 19K are both laminated type piezoelectric elements and are juxtaposed forwards and rearwards in parallel relation to each other so as to assume a parallel relation with the main frame portion 73 a of the stationary pedestal 73 along the direction of lamination thereof. Those two sets of the piezoelectric elements laminates 19J and 19K are connected in series with each other by means of a fastening member 78. The fastening member 78 is of a generally Z-shaped configuration including a lengthwise segment 78a disposed between the piezoelectric elements laminates 19J and 19K, positioned forwards and rearwards, respectively, in parallel relation with the piezoelectric elements laminates 19J and 19K, and projecting segments 78b and 78c situated at both ends of the lengthwise segment 78a and projecting in a forward and rearward direction so as to assume an inverted relation to each other. The set of the piezoelectric elements laminate 19J is supported at one end by the side frame portion 73 c of the stationary pedestal 73 and is connected at the opposite end with the projecting segment 78b of the fastening member 78 which is oriented towards the expansion and contraction direction movable body 74. Also, the remaining set of the piezoelectric elements laminate 19K is connected at one end with the projecting segment 78c of the fastening member 78, which is oriented towards the stationary pedestal side frame portion 73c, and at the opposite end with the expansion and contraction direction movable body 74. The spring member 27D referred to previously applies a preload to the piezoelectric elements laminate 19K. Accordingly, the expansion and contraction direction movable body 74 is displaceable in the X-axis direction depending on an expanding or contracting displacement of the piezoelectric elements laminates 19J and 19K.

In this Y-axis transfer mechanism 15, there is provided a link mechanism 75 which functions as an amplifying mechanism for amplifying the expansion and contraction of the piezoelectric elements laminates 19J and 19K to a displacement in a direction (Y-axis direction) perpendicular to the expansion and contraction direction (X-axis direction) thereof. This link mechanism 75 is connected to the stationary pedestal side frame portion 73c with the use of one fixing joint 76 and also connected to the expansion and contraction direction movable body 74 with the use of one movable joint 77A.

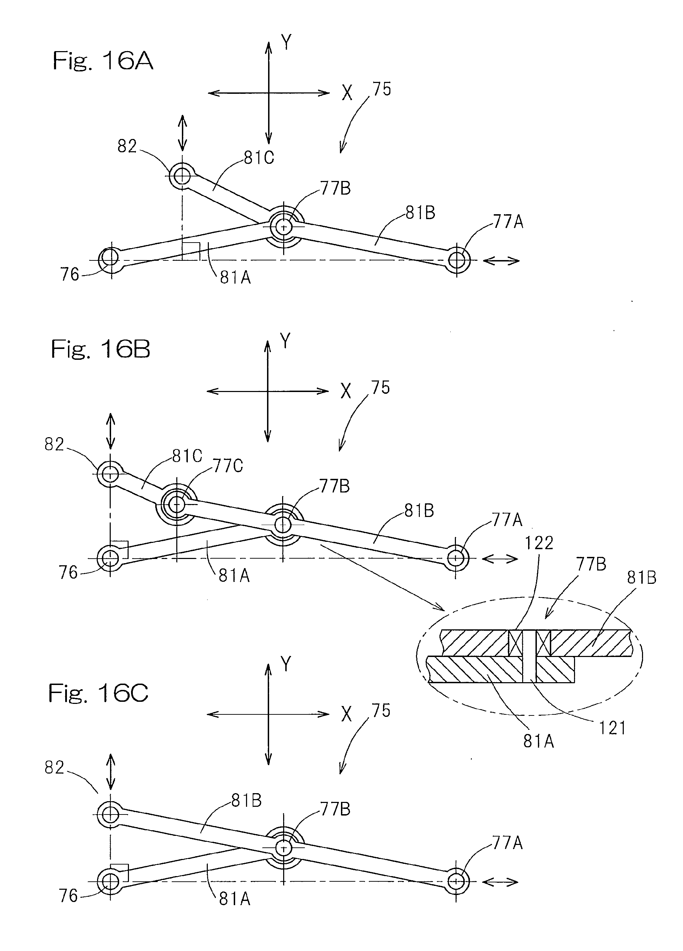

Figs. 16A to 16C illustrate different examples of the structure of the link mechanism 75. In the example of the structure shown in Fig. 16A, the link mechanism 75 is comprised of one fixed joint 76, two movable joints 77A and 77B and three links 81A, 81B and 81C.

Each of the fixed joint 76 and movable joint 77A and 77B is a pivotable joint forming a nodal point and the center point of pivotal movement thereof lies orthogonal to any one of the expansion and contraction direction (X-axis direction) of the piezoelectric elements laminates 19J and 19K and the direction (Y-axis direction) perpendicular to such expansion and contraction direction. This description equally applies to any one of the joints 76, 77A, 77B and 77C shown in Figs. 16B and 16C.

The first and second links 81A and 81B are connected at one end with the fixed joint 76 and the first movable joint 77A, respectively, and at the opposite end with the second movable joint 77B. The third link 81C is connected at one end with the second movable joint 77B and has the opposite end forming a movable segment 82 that is constrained by a guide mechanism (not shown) such as, for example, a linear guide or a direct acting bearing for movement only in a direction (Y-axis direction) perpendicular to the expansion and contraction direction. The fixed joint 76 is fixed in position relative to the side frame portion 73c of the stationary pedestal 73 to which one end of the piezoelectric elements laminates 19J and 19K on a fixed side is fixed. The guide mechanism for guiding the movable segment 82 is provided in this stationary pedestal 73. The first movable joint 77A is provided in the expansion and contraction movable body 74 movable together with one end of the piezoelectric elements laminates 19J and 19K on a movable side and is movable together with this expansion and contraction direction movable body 74. The movable segment 82 of the third link 81C forms a displacement amplifying output unit of the link mechanism 75.

According to the above described construction, the first movable joint 77A displaces in the X-axis direction in response to expansion or contraction of the piezoelectric element laminates 19J and 19K and, with this displacement amplified, the second movable joint 77B displaces in the Y-axis direction. When the third link 81C changes its angle of pivot incident thereto, the movable segment 82 at the other end of the third link 81C displaces having been guided by the guide mechanism such as, for example, the linear guide.

In such case, the frictional resistance is reduced when a rolling bearing is employed in each of the fixed joint 76 and movable joints 77A and 77B and, when a proper preload is applied to such rolling bearing, rattling is suppressed to allow a precise positioning to be realized. Also, since there is no elastic deforming portion, designing is easy to achieve.

In the example of the structure shown in Fig. 16B, the link mechanism 75 is comprised of one fixed joint 76, three movable joints 77A, 77B and 77C and three links 81A, 81B and 81C. This example of the structure shown in Fig. 16B is such that in the example of the structure shown in and described with reference to Fig. 16A, the movable joint connected with a base end of the third link 81C is rendered to be the third movable joint 77C at a position different from that of the second movable joint 77B.

In other words, the other end of the first link 81A having a base end connected with the fixed joint 76 is connected with an intermediate portion of the second link 81B, having a base end connected with the first movable joint 77A, through the second movable joint 77B. The third link 81C is connected with a tip end of the second link 81B through the third movable joint 77C. A tip end of the third link 81 is rendered to be a movable segment 82 so constrained by a guide mechanism (not shown), which is similar to that described hereinbefore, for movement only in one direction (X-axis direction) perpendicular to the expansion and contraction direction (Y-axis direction). The fixed joint 76 and the first movable joint 77a are provided in the stationary pedestal 73 and the expansion and contraction direction movable body 74, respectively, in a manner similar to that in the example of the structure shown in and described with reference to Fig. 16A. In the example of the structure shown in Fig. 16B, the movable segment 82 of the tip end of the third link 81C becomes a displacement amplifying output unit for the link mechanism 75.

It is to be noted that the second movable joint 77B is, for example, of such a structure that, as shown in Fig. 16B on an enlarged scale, one of the first and second links 81A and 81B is connected through a bearing 122 with a connecting pin 121 fixed to the other of the first and second links 81A and 81B. The bearing 122 is engaged in a hole defined in one of the links 81A and 81B. This bearing 122 may be either a rolling bearing such as, for example, a ball bearing or a slide bearing, but is chosen in the form of, for example, a rolling bearing capable of applying a preload.

According to the above described construction, when expansion or contraction of the piezoelectric elements laminates 19J and 19K results in a displacement of the movable joint 77A in the X-axis direction and this displacement is amplified and brings about a displacement of the different movable joint 77C in the Y-axis direction to cause the third link 81C to change its angle of pivot, the movable segment 82 of the tip end of the third link 81C displaces in the Y-axis direction having been guided by the guide mechanism.

Any of the examples of the structures shown in and described with reference to Figs. 16A and 16B, respectively, is effective in that even though any influence occurs as a result of a dimensional error and/or thermal deformation of each of the links 81A, 81B and 81C, it can be absorbed because the angle of the third link 81C is independently variable and the movable segment 82 due to function as the displacement amplifying output unit can move in a linear direction in the Y-axis direction along the guide mechanism such as, for example, the previously described linear guide.

It is to be noted that in the example of the structure shown in and described with reference to Fig. 16A, three links 81A, 81B and 81C are connected at the position of the second movable joint 77B and, therefore, the two bearings are necessarily juxtaposed in the axial direction thereof, resulting in an increase of the dimension in the direction of thickness. However, in the case of the example of the structure shown in and described with reference to Fig. 16B, only one bearing 122 is required in the movable joints 77B and 77C and, therefore, the thicknesswise dimension can be reduced to 1/2 of that in the example of the structure shown in and described with reference to Fig. 16A.

In the example of the structure show in Fig. 16C, the link mechanism 75 is comprised of one fixed joint 76, two movable joint 77A and 77B and two links 81A and 81B.

In other words, the other end of the first link 81A having its base end connected with the fixed joint 76 is connected through the second movable joint 77B with an intermediate portion of the second link 81B having a base end connected with the first movable joint 77A. In a manner similar to each of the examples of the structure shown in and described with reference to Figs. 16A and 16B, respectively, the fixed joint 77B is connected with the side frame portion 73c of the stationary pedestal 73 and the first movable joint 77A is connected with the expansion and contraction direction movable body 74. In this example of the structure, the movable segment 82 at the tip end of the second link 81B constitutes a displacement amplifying output unit for the link mechanism 75.

In the case of the above described construction, since the length of the second link 81B is twice that of the first link 81A and the second movable joint 77B is arranged at an intermediate position of the link 81B, the direction of movement of the movable segment 82 is constrained without the guide mechanism such as the linear guide being employed. The direction of movement thereof is rendered to be displaceable in a direction at right angles to the straight line drawn between the fixed joint 76 and the center of the movable joint 77A, that is, a direction (Y-axis direction) perpendicular to the expansion and contraction direction (X-axis direction) of the piezoelectric elements laminates 19J and 19K.

According to the above described construction, the movable joint 77A displaces in the X-axis direction as a result of expansion and contraction of the piezoelectric elements laminates 19J and 19K, and this displacement is amplified and the movable segment 82, which is a tip end of the second link 81B, displaces in the Y-axis direction. This example of the structure becomes the most compact one. Even in this example, only one bearing is needed in this movable joint 77B in its axis direction and, as compared with the example of the structure shown in and described with reference to Fig. 16A, the thicknesswise dimension can be reduced to 1/2.

In the Y-axis transfer mechanism 15 shown in Fig. 15, the link mechanism 75 in the example of the structure shown in and described with reference to Fig. 16C is employed as the amplifying mechanism. Other structural features are similar to those employed in the Y-axis transfer mechanism 15 shown in and described with reference to Fig. 13.

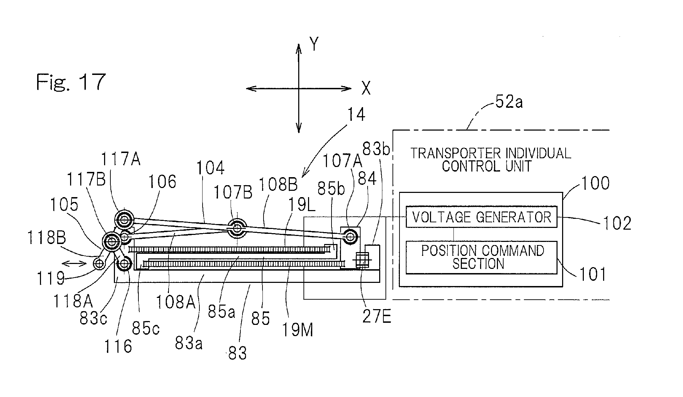

Fig. 17 illustrates another example of the structure of the X-axis transfer mechanism 14. Even in this example of the structure, a stationary pedestal 83, and two sets of piezoelectric elements laminates 19L and 19M functioning as a drive source are employed in a manner similar to the Y-axis transfer mechanism 15 shown in Fig. 15. The stationary pedestal 83 includes a main frame portion 83 a, extending in the X-axis direction, and a pair of side frame portions 83b and 83d both extending from opposite ends of the main frame portion 83a in a widthwise direction (Y-axis direction), A movable segment 84, confronting the side frame portion 83c at one end the stationary pedestal 83, is supported by the side frame portion 83b at the opposite end of the stationary pedestal 83 for movement in the X-axis direction through a spring member 27E.

Two sets of piezoelectric elements laminates 19L and 19M are both laminated type piezoelectric elements and are juxtaposed forwards and rearwards in parallel relation to each other so as to assume a parallel relation with the main frame portion 83a of the stationary pedestal 83 along the direction of lamination thereof. Those two sets of the piezoelectric elements laminates 19L and 19M are connected in series with each other by means of a fastening member 85. The fastening member 85 is of a generally Z-shaped configuration including a lengthwise segment 85a disposed between the piezoelectric elements laminates 19L and 19M, positioned forwards and rearwards, respectively, in parallel relation with the piezoelectric elements laminates 19L and 19M, and projecting segments 85b and 85c situated at both ends of the lengthwise segment 85a and projecting in a forward and rearward direction so as to assume an inverted relation to each other. The set of the piezoelectric elements laminate 19L is supported at one end by the side frame portion 83c of the stationary pedestal 83 and is connected at the opposite end with the projecting segment 85b of the fastening member 85 which is oriented towards the movable segment 84. Also, the remaining set of the piezoelectric elements laminate 19M is connected at one end with the projecting segment 85c of the fastening member 85, which is oriented towards the stationary pedestal side frame portion 83c, and at the opposite end with the movable segment 84. The spring member 27E referred to previously applies a preload to the piezoelectric elements laminate 19M. Accordingly, the movable segment 84 is displaceable in the X-axis direction depending on an expanding or contracting displacement of the piezoelectric elements laminates 19L and 19M.

In this X-axis transfer mechanism 14, a first link mechanism 104 is employed as a first amplifying mechanism for amplifying the expansion and contraction of the piezoelectric elements laminates 19L and 19M into a displacement in a direction (X-axis direction) of expansion or contraction thereof and a direction (Y-axis direction) perpendicular thereto and, also, a second link mechanism 105 is employed as a second amplifying mechanism for amplifying the displacement, which is amplified by the first amplifying mechanism (first link mechanism 104) into a displacement of the piezoelectric elements laminates 19L and 19M in the direction of expansion or contraction. For each of the link mechanisms 104 and 105, the same link mechanism as the link mechanism 75 shown in and described with reference to Fig. 16C is employed. It is to be noted that the second link mechanism 105 is rendered to have an attitude that is inverted 90° in direction relative to the first link mechanism 104.

In other words, the first link mechanism 104 is comprised of one fixed joint 106, two movable joints 107A and 107B and two links 108A and 108B. More specifically, the first link 108A has one end connected with the side frame portion 83c of the stationary pedestal 83 through the fixed joint 106, and the second link 108B has one end connected through the movable joint 107A with the movable segment 84 which is displaceable in the direction of expansion or contraction in response to expansion or contraction of the piezoelectric elements laminates 19L and 19M, and an intermediate portion connected with the other end of the first link 108A through another movable joint 107B. The second link 108B has the opposite end which is displaceable in the direction (Y-axis direction) perpendicular to the direction (X-axis direction) of expansion or retraction of the piezoelectric elements laminates 19L and 19M.

The second link mechanism 105 is similarly comprised of one fixed joint 116, two movable joints 117A and 117B and two links 118A and 118B. More specifically, the first link 118A has one end connected with the side frame portion 83c of the stationary pedestal 83 through the fixed joint 116, and the second link 118 has one end connected through the movable joint 117A with the other end of the second link 108B of the first link mechanism 104 that is displaceable in the direction (Y-axis direction) perpendicular to the direction of expansion or contraction in response to expansion or contraction of the piezoelectric elements laminates 19L and 19M, and also an intermediate portion connected with the other end of the first link 118A through another movable joint 117B. Accordingly, the first and second link mechanisms 104 and 105 are assembled in two stages on the same plane through the movable joint 117A referred to above. In such case, a tip end of the second link 118B in the second link mechanism 105 forms a movable segment 119 which is a displacement amplifying output unit and is rendered to be displaceable in the direction (X-axis direction) of expansion or contraction of the piezoelectric elements laminates 19L and 19M.

According to the above described construction, in the first link mechanism 104, the movable joint 107 displaces in the X-axis direction in response to expansion and contraction of the piezoelectric elements laminates 19L and 19M and, after this displacement has been amplified, the movable joint 117A, which is the other end of the link 108B, displaces in the Y-axis direction. Also, in the second link mechanism 105, with the displacement of the movable joint 117A in the Y-axis direction having been amplified, the movable segment 119, which is the other end of the link 118B, displaces in the direction (X-axis direction) of expansion and contraction of the piezoelectric elements laminates 19L and 19M. Other structural features are similar to those in the X-axis transfer mechanism 14 shown in and described with reference to Fig. 14.

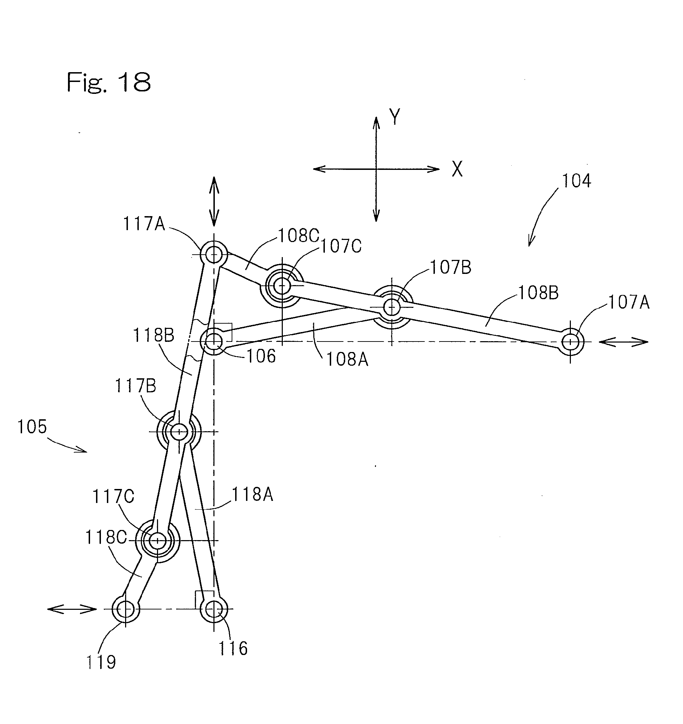

It is to be noted that in the X-axis transfer mechanism 14 shown in and described with reference to Fig. 17, for the first and second link mechanisms 104 and 105, what has been shown and described in connection with the example of the structure shown in Fig. 16C is assembled in two stages through the movable joint 117A, but the present invention may not be necessarily limited thereto and what has been employed in the example of the structure shown in and described with reference to Fig. 16B may be assembled in two stages through the movable joint, an example of which is shown in Fig. 18. In this example shown in Fig. 18, the second link mechanism 105 is shown as having an attitude inverted 90° in direction relative to the first link mechanism 104.

In the example shown in Fig .18, the first link mechanism 104 is made up of one fixed joint 106, three movable joints 107A, 107B and 107C, and two links 108A and 108B. The second link mechanism 105 is made up of one fixed joint 116, three movable joints 117A, 117B and 117C and two links 118A and 118B. By connecting the movable segment in the first link mechanism 104 with a base end of the second link 118B in the second link mechanism 105 through the movable joint 117A, the first and second link mechanisms 104 and 105 are assembled in two stages on the same plane. A tip end of the second link 118B of the second link mechanism 105 becomes a movable segment 119, which is a displacement amplifying output unit, and is rendered to be displaceable in the direction (X-axis direction) of expansion and contraction of the piezoelectric elements laminates 19L and 19M.

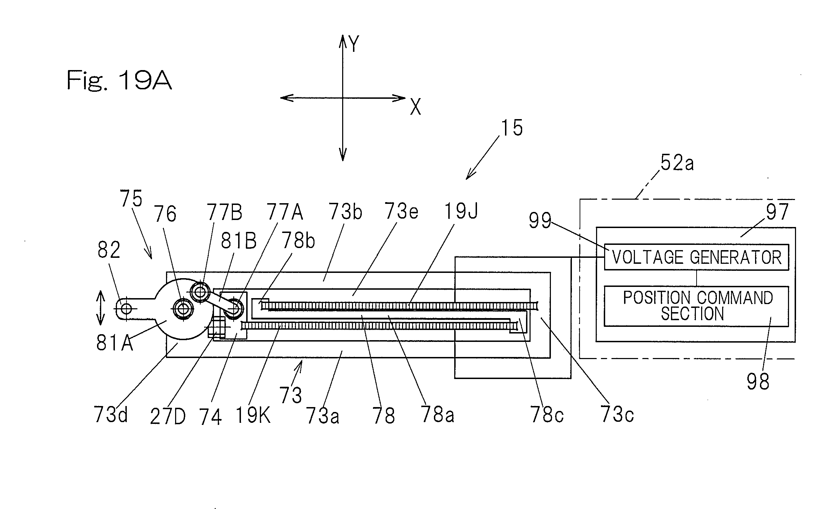

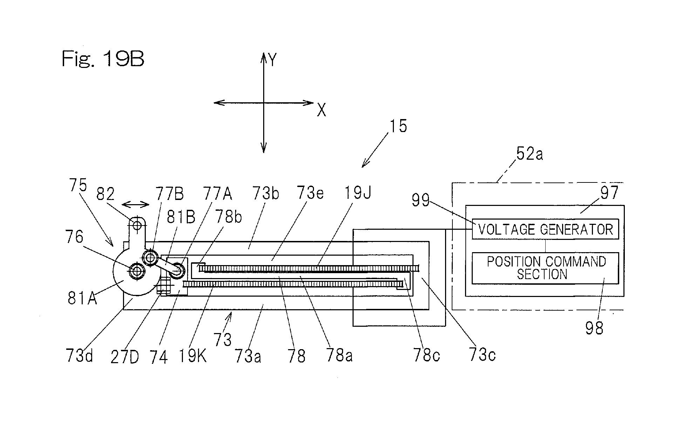

As a different example of the structure of the link mechanism 75 referred to previously, as shown in Figs. 19A and 19B, the link mechanism 75 include a cranking and sliding mechanism, which is made up of one fixed joint 76, two movable joints 77A and 77B and two links 81A and 81B, and this cranking and sliding mechanism may be designed to have a capability of converting and amplifying a displacement of the piezoelectric elements in a direction of extension thereof into an arbitrary direction on the circumference of the fixed joint 76 through the two movable joints 77A and 77B and the two links 81A and 81B.

Fig. 19A illustrates a further example of the structure of the Y-axis transfer mechanism 15. Even the Y-axis transfer mechanism 15 according to this further example of the structure shown in Fig. 19A is made up of a stationary pedestal 73 and two sets of piezoelectric elements laminates 19J and 19K functioning as a drive source as is the case with the example of the structure shown in and described with reference to Fig. 15. The stationary pedestal 73 includes a pair of main frame portions 73a and 73b extending in the X-axis direction, a pair of side frame portions 73c and 73d extending from opposite ends of the main frame portions 73a and 73b in a widthwise direction (Y-axis direction), and a side plate 73 e for fastening four frame portions. An expansion and contraction direction movable body 74 confronting the side frame portion 73c at one end of the stationary pedestal 73 is supported by the side frame portion 73d at the other end of the stationary pedestal 73 for movement in the X-axis direction through a spring member 27D.

The two sets of the piezoelectric elements laminates 19J and 19K are both laminated type piezoelectric elements and are juxtaposed forwards and rearwards in parallel relation to each other so as to assume a parallel relation with the main frame portions 73a and 73b of the stationary pedestal 73 along the direction of lamination thereof. Those two sets of the piezoelectric elements laminates 19J and 19K are connected in series with each other by means of a fastening member 78. The fastening member 78 is of a generally Z-shaped configuration including a lengthwise segment 78a disposed between the piezoelectric elements laminates 19J and 19K, positioned forwards and rearwards, respectively, in parallel relation with the piezoelectric elements laminates 19J and 19K, and projecting segments 78b and 78c situated at both ends of the lengthwise segment 78a and projecting in a forward and rearward direction so as to assume an inverted relation to each other. The set of the piezoelectric elements laminate 19J is supported at one end by the side frame portion 73c of the stationary pedestal 73 and is connected at the opposite end with the projecting segment 78b of the fastening member 78 which is oriented towards the expansion and contraction direction movable body 74. Also, the remaining set of the piezoelectric elements laminate 19K is connected at one end with the projecting segment 78c of the fastening member 78, which is oriented towards the stationary pedestal side frame portion 73c, and at the opposite end with the expansion and contraction direction movable body 74. The spring member 27D referred to previously applies a preload to the piezoelectric elements laminate 19K. Accordingly, the expansion and contraction direction movable body 74 is displaceable in the X-axis direction depending on the expansion or contraction of the piezoelectric elements laminates 19J and 19K.

The Y-axis transfer mechanism 15 of the structure described above is provided with the link mechanism 75 as an amplifying mechanism for amplifying the expansion and contraction of the piezoelectric elements laminates 19J and 19K to a displacement in a direction (Y-axis direction) perpendicular to the expansion and contraction direction (X-axis direction) thereof. This link mechanism 75 is connected to the stationary pedestal side frame portion 73d with the use of one fixing joint 76 and also with the expansion and contraction direction movable body 74 with the use of one movable joint 77A.

According to the above described construction, the movable joint 77A displaces in the X-axis direction as a result of expansion or contraction of the piezoelectric elements laminates 19J and 19K and the movable joint 77B undergoes a pivotal movement about the fixed joint 76. Therefore, the movable segment 82 fixed to the first link 81A displaces in the Y-axis direction. Accordingly, the displacement can be amplified in an arbitrary direction by setting the output unit at an arbitrary position on the circumference of the fixed joint 76. Hence, the number of component parts and the dimension of the Y-axis transfer mechanism 15 can be reduced.

Fig. 19B illustrates a further example of the structure of the X-axis transfer mechanism 14. Even the X-axis transfer mechanism 14 shown in Fig. 19B has a structure similar to that shown in and described with reference to Fig. 19A, and by changing the positions of the movable segment 82 and the movable joint 77B fixed to the link 81A, it displaces in the X-axis direction. Accordingly, the displacement can be amplified in an arbitrary direction by setting the output unit at an arbitrary position on the circumference of the fixed joint 76 and, hence, the number of component parts and the dimension of the Y-axis transfer mechanism 15 can be reduced.

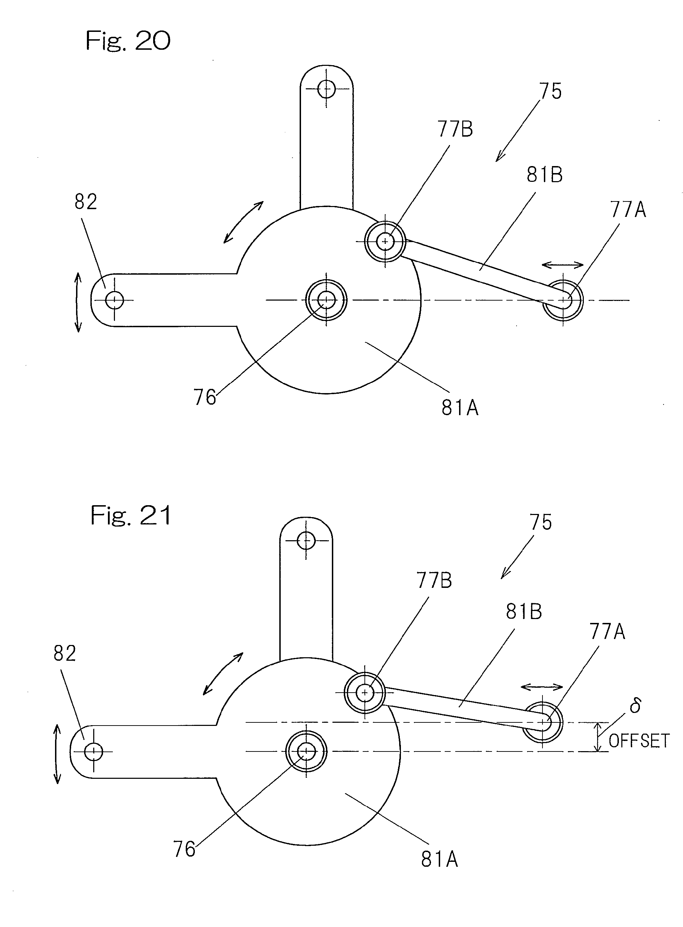

Fig. 20 illustrates, on an enlarged scale, the example of the structure of the link mechanism 75 shown in and described with reference to Figs. 19A and 19B.

In the example of the structure of the link mechanism 75 shown in and described with reference to Figs. 19A and 19B, the fixed joint 76 and the movable joints 77A and 77B, which form the link mechanism 75 that will become a cranking and sliding mechanism comprised of the one fixed joint 76, the two movable joints 77A and 77B and the two links 81A and 81B, are pivotable joints forming respective nodal points and the center point of pivotal movement thereof lies at right angles to any one of the expansion and contraction direction (X-axis direction) of the piezoelectric elements laminates 19J and 19K and the direction (Y-axis direction) perpendicular to such expansion and contraction direction.

The first and second links 81A and 81B are connected at one end with the fixed joint 76 and the first movable joint 77A, respectively, and at the opposite end with the second movable joint 77B. The first movable joint 77A is provided in the expansion and contraction direction movable body 74 (best shown in Figs. 19A and 19B) capable of moving together with the end of the piezoelectric elements laminates 19J and 19K on an expansion side and is rendered to be movable together with this expansion and contraction direction movable body 74.

Fig. 21 illustrates the structure of an offset cranking mechanism in which the fixed joint 76 does not lie on the line of extension of a sliding direction of the first movable joint 77A and hence has an offset. An effect of the offset will now be described with particular reference to Fig. 22. Fig. 22 illustrates the amount of movement of the first movable joint 77A when the link 81A is caused to undergo one complete rotation about the fixed joint 76. The solid line in Fig. 22 shows the case with no offset, in which relative to a reciprocal movement of the movable joint 77A, the link 81A rotates about the fixed joint 76, the angle of rotation of which is 180° in the event that the movable joint 77A moves a maximum distance in a leftwards direction as viewed in the drawing, and the relation between the angle of rotation of the link 81A and the amount of movement of the movable joint 77A is symmetrical with respect to the angle of 180°. In contrast thereto, the broken line in Fig. 22 shows the case with offset shown in Fig. 21, in which relative to the reciprocal movement of the movable joint 77A, the angle of rotation of the link 81A becomes large in the event that the movable joint 77B lies on an offset side (upper area in the drawing).

Where the displacement of the movable joint 77A of several hundreds µm in amount of movement is amplified to several millimeters such as in the case of the present invention, the angle of rotation of the link 81A will be about several degrees. In general the displacement amplifying rate is determined by the length of each of the first and second links 81A and 81B, the initial position of the movable joint 77A and the length between the movable segment 82 and the fixed joint 76, but if compactization of the device is considered, a further increase of the amplifying rate can be expected provided that as is the case with the present invention, the offset δ is employed without altering the length of the link and the angle of rotation of the link 81A relative to the amount of movement of the movable joint 77a is set to a phase where such angle of rotation becomes large.

In particular, in the microinjection apparatus, since the transporter 4 includes the transfer mechanisms (the X-axis transfer mechanism 14, the Y-axis transfer mechanism 15 and the Z-axis transfer mechanism 16) of three degrees of freedom and each of those transfer mechanisms 14 to 16 makes use of the laminated piezoelectric elements (piezoelectric elements laminates 19D to 19M), further effects can be obtained that compactization of the transporter 4 has now become possible, the plurality of the transporters 4 can be arranged around the container 12 such as, for example, petri dish accommodating therein the transductant, and, hence, the injecting operations into the plurality of the cells can be carried out all at a time. It is to be noted that if the transporter 4 includes the transfer mechanism of at least two degrees of freedom and the laminated piezoelectric elements are used as a drive source for at least one degree of freedom out of the transfer mechanisms of the respective degrees of freedom, the transporter 4 can have a reduced size. Considering that the piezoelectric element has a high efficiency of conversion of electric energies into mechanical energies, if the voltage to be applied is changed, the resultant displacement can be relatively easily made variable, accompanied by an excellent controllability.

As hereinbefore described, in order for the tip end of the injection needle 11 to be movable over the entire region of the predetermined field of view in the image obtained by the imaging device 2, it is preferred that the distance of movement of the injection needle 11 caused by the transporter 4 is not smaller than 1 mm. Since in the embodiment hereinbefore described, as drive source for the transfer mechanisms 14, 15 and 16 in the transporter 4, the two set of the laminated type piezoelectric elements (the piezoelectric elements laminates 19H and 19I or 19L and 19M in the case of the X-axis transfer mechanism 14; the piezoelectric elements laminates 19F and 19G or 19J and 19K in the case of the Y-axis transfer mechanism 15; and the piezoelectric elements laminates 19D and 19E in the case of the Z-axis transfer mechanism 16) are arranged parallel to each other in a direction along the direction of lamination of the piezoelectric elements and those are connected in series with each other by means of the fastening members 48, 58, 78, 85 and 103, a required amount of movement of the injection needle 11 can be sufficiently secured with no need to use a large space.

Also, since in the embodiment hereinbefore described, the X-axis transfer mechanism 14 and the Y-axis transfer mechanism 15 in the transporter 4 are configured to have the amplifying mechanism for amplifying the displacement of each of the piezoelectric elements laminates (the piezoelectric elements laminates 19H and 19I or 19L and 19M in the X-axis transfer mechanism 14 and the piezoelectric elements laminates 19F and 19G or 19J and 19K in the Y-axis transfer mechanism 15) into the displacement in the direction (Y-axis direction) perpendicular to the direction of such displacement (X-axis direction), it is indeed effective for securing the required amount of movement of the injection needle 11.

Moreover, since in the embodiment hereinbefore described, the X-axis transfer mechanism 14 and the Y-axis transfer mechanism 15 in the transporter 4 are configured to have the first amplifying mechanism for amplifying the displacement of each of the piezoelectric elements laminates (the piezoelectric elements laminates 19H and 19I or 19L and 19M in the X-axis transfer mechanism 14 and the piezoelectric elements laminates 19F and 19G or 19J and 19K in the Y-axis transfer mechanism 15) into the displacement in the direction perpendicular to the direction of such displacement and the second amplifying mechanism for amplifying the displacement, so amplified by the first amplifying mechanism, into the displacement in the displacement direction (X-axis direction) of the piezoelectric elements laminate, it is further effective for securing the required amount of movement of the injection needle 11.

Furthermore, in the embodiment hereinbefore described, as shown in Fig. 11, the Z-axis transfer mechanism 16 for moving the needle support member 18 for supporting the injection needle 11 in a direction of insertion (Z-axis direction) of the injection needle 11 is provided with the vibration driver 93 for vibrating the needle support member 18 in the direction of insertion by repeatedly changing the displacement of a part of the piezoelectric elements laminate 19E1 in the piezoelectric elements laminates 19D and 19E functioning as a drive source thereof and the piezoelectric elements laminates 19D and 19E are separately utilized for vibration purpose and for positioning purpose, respectively. For this reason, a smooth piercing of the injection needle 11 into the cell, which forms the transductant, can be enabled and a high speed drive for positing of the injection needle 11 can be realized.

Since in the example of the structure of the z-axis transfer mechanism 16 shown in Fig. 12, the vibration driver 93 is so configured as to apply to that part of the piezoelectric elements laminate 19E1, a signal in which the positioning signal used to reposition the needle support member 18 and the vibration drive signal used to vibrate the needle support member 18 in the direction of insertion are superimposed with each other, that part of the piezoelectric element laminate 19E1 can be concurrently employed for vibration application and also for positioning.

Yet, since in the embodiment hereinabove described, that part of the piezoelectric elements laminate 19E1 contributing to the application of the vibration is connected directly with the needle support member 18, the displacement for the vibration to be generated in the piezoelectric elements laminate 19E1 can be efficiently transmitted to the injection needle 11.

Furthermore, since in the embodiment hereinabove described, the vibration driver 93 of the Z-axis transfer mechanism 16 is designed to have the frequency changer 94 for switching the frequency of vibration, the frequency of the vibration referred to above can be altered depending on the kind, cell membrane and nuclear membrane of the cell which forms the transductant into which the injection needle 11 is inserted, and, therefore, a smooth insertion of the injection needle 11 can be performed.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. Accordingly, such changes and modifications are, unless they depart from the scope of the present invention as delivered from the claims annexed hereto, to be construed as included therein.

### [Reference Numerals]

- 1: Container position adjuster
- 2: Imaging device
- 3: Position determiner
- 4: Transporter
- 5: Control device
- 11: Injection needle
- 12: Container
- 14: X-axis transfer mechanism
- 15: Y-axis transfer mechanism
- 16: Z-axis transfer mechanism
- 19A to 19M: Piezoelectric element laminate
- 26: Movable piece (Amplifying mechanism)
- 30: Movable piece (Amplifying mechanism)
- 32: Movable frame portion (Amplifying mechanism)
- 33: Transporter retracting mechanism
- 48, 58, 78, 85, 103: Fastening member
- 50: Controller
- 51: Assigned transductant determining unit
- 52: Injecting operation control unit
- 75: Link mechanism (Amplifying mechanism)
- 76: Fixed joint
- 77A, 77B: Movable joint
- 81A to 81C: Link
- 93: Vibration driver
- 104: First link mechanism (First amplifying mechanism)
- 105: Second link mechanism (Second amplifying mechanism)
- 106: Fixed joint
- 107A, 107B: Movable joint
- 108A, 108B: Link

## Claims

1. A microinjection apparatus for introducing a transfecting material into a transductant by inserting a minute injection needle into the transductant, the injection needle being filled with the transfecting material therein, which apparatus comprises:
a container position adjuster for adjusting a position of a container accommodating therein the transductant; and
a plurality of transporters for individually moving a plurality of injection needles to the transductant within the corresponding container, of which position has been adjusted by the container position adjuster;
an imaging device for capturing through a magnifying lens, an image of an inside of the container, of which position has been adjusted by the container position adjuster;
a position determiner for determining a position of the transductant from the image obtained by the imaging device; and
a controller for causing each of the transporters to move the injection needle in dependence on a position information obtained by the position determiner.

2. The microinjection apparatus as claimed in claim 1, in which the plurality of the transporters are arranged in a radial pattern around the container.

3. The microinjection apparatus as claimed in claim 1, in which the controller is so configured as to recognize respective positions of a plurality of transductants within the container according to the position information obtained by the position determiner and as to actuate the plurality of the transporters parallel to insert a plurality of injection needles into the plural transductants.

4. The microinjection apparatus as claimed in claim 1, in which the controller is so configured as to recognize a position of the transductant within the container according to the position information obtained by the position determiner and as to successively actuate the plurality of the transporters to insert a plurality of the injection needles into the same transductant.

5. The microinjection apparatus as claimed in claim 1 further comprising a transporter retracting mechanism for advancing or retracting each of the transporters between a ready-to-inject position, at which a tip end of the injection needle approaches the transductant, and a retracted position.

6. The microinjection apparatus as claimed in claim 1, in which the transductant is a cell and the transfecting material is a gene regulatory factor.

7. The microinjection apparatus as claimed in claim 1, in which each of the transporters has at least two degrees of freedom and transfer mechanisms are provided one for each degree of freedom, and in which at least one of the transfer mechanisms has a drive source employed in the form of a piezoelectric elements laminate comprised of a plurality of piezoelectric elements laminated and capable of expanding or contracting in a direction of lamination thereof.

8. The microinjection apparatus as claimed in claim 7, in which the transfer mechanism in each of the transporters, the drive source of which is employed in the form of the piezoelectric elements laminate, is of a structure in which a plurality of piezoelectric elements laminates are arranged parallel to each other and connected in series with each other in a direction conforming to expansion or contraction thereof by means of a fastening member.

9. The microinjection apparatus as claimed in claim 7, in which the transfer mechanism in each of the transporters, the drive source of which is employed in the form of the piezoelectric elements laminate, includes an amplifying mechanism for amplifying a displacement of the expansion or contraction of the piezoelectric elements laminate in a direction perpendicular to a direction of expansion or contraction of the piezoelectric elements laminate.

10. The microinjection apparatus as claimed in claim 9, in which the amplifying mechanism comprises a link mechanism that is made up of first, second and third links, one fixed joint fixed in position and first, second and third movable joints each movable in position, each of those joints being a pivotable joint forming a nodal point and having a center of pivot lying perpendicular to any one of the direction of expansion and contraction of the piezoelectric elements laminate and the direction perpendicular to the direction of expansion and contraction;
in which the first link having a base end connected with the fixed joint has the other end connected with an intermediate portion of the second link, having a base end connected with the first movable joint, through the second movable joint, the third link is connected with a tip end of the second link through the third movable joint, and the third link has a tip end rendered to be a movable segment which is constrained for movement only in the direction perpendicular to expansion and contraction; and
in which the fixed joint is fixed in position relative to an end of the piezoelectric elements laminate on a fixed side, the first movable joint is made movable together with an end of the piezoelectric elements laminate on an expansion side, and the movable segment at the tip end of the third link is rendered to be a displacement amplifying output unit of the link mechanism.

11. The microinjection apparatus as claimed in claim 9, in which the amplifying mechanism comprises a link mechanism that is made up of first and second links, a fixed joint fixed in position and first and second movable joints movable in position, each of those joints being a pivotable joint forming a nodal point and having a center of pivot lying perpendicular to any one of the direction of expansion and contraction of the piezoelectric elements laminate and the direction perpendicular to the direction of expansion and contraction;
in which the first link having a base end connected with the fixed joint has the other end connected with an intermediate portion of the second link, which has a base end connected with the first movable joint, through the second movable joint; and
in which the fixed joint is fixed in position relative to an end of the piezoelectric elements laminate on a fixed side, the first movable joint is rendered to be movable together with an end of the piezoelectric elements laminate on an expansion side, and the second link has a tip end which will become a displacement amplifying unit of the link mechanism.

12. The microinjection apparatus as claimed in claim 7, in which the transfer mechanism in each of the transporters, the drive source of which is employed in the form of the piezoelectric elements laminate, includes a first amplifying mechanism for amplifying the expansion or contraction of the piezoelectric element into a displacement in a direction perpendicular to the direction of expansion or contraction and a second amplifying mechanism for amplifying the displacement, which has been amplified by the first amplifying mechanism, into a displacement in the direction of expansion or contraction of the piezoelectric element.

13. The microinjection apparatus as claimed in claim 12, in which each of the first and second amplifying mechanisms comprises a link mechanism that is made up of first, second and third links, a fixed joint fixed in position and first, second and third movable joints each movable in position, each of those joints being a pivotable joint forming a nodal point and having a center of pivot lying perpendicular to any one of the direction of expansion and contraction of the piezoelectric elements laminate and the direction perpendicular to the direction of expansion and contraction;
in which the first link having a base end connected with the fixed joint has the other end connected with an intermediate portion of the second link, having a base end connected with the first movable joint, through the second movable joint; the third link is connected with a tip end of the second link through the third movable joint, and the third link has a tip end rendered to be a movable segment which is constrained for movement only in the direction perpendicular to expansion and contraction;
in which the fixed joint in the link mechanism forming the first amplifying mechanism is fixed in position relative to an end of the piezoelectric elements laminate on a fixed side, and the first movable joint is made movable together with an end of the piezoelectric elements laminate on an expansion side;
in which the link mechanism forming the second amplifying mechanism is provided in such an attitude that the link mechanism forming the first amplifying mechanism is altered 90° about a center axis parallel to an axis of pivot of each of the joints, and the fixed joint is fixed in position relative to an end of the piezoelectric elements laminate on a fixed side and the first movable joint is provided in the movable segment in the link mechanism forming the first amplifying mechanism; and
in which the movable segment of the third movable joint in the link mechanism forming the second amplifying mechanism is rendered to be a displacement amplifying output unit of the link mechanism.

14. The microinjection apparatus as claimed in claim 12, in which each of the first and second amplifying mechanisms comprises a link mechanism that is made up of first and second links, a fixed joint fixed in position and first and second movable joints movable in position, each of those joints being a pivotable joint forming a nodal point and having a center of pivot lying perpendicular to any one of the direction of expansion and contraction of the piezoelectric elements laminate and the direction perpendicular to the direction of expansion and contraction;
the first link having a base end connected with the fixed joint has the other end connected with an intermediate portion of the second link, which has a base end connected with the first movable joint, through the second movable joint;
the fixed joint in the link mechanism forming the first amplifying mechanism is fixed in position relative to an end of the piezoelectric elements laminate on a fixed side, and the first movable joint is rendered to be movable together with an end of the piezoelectric elements laminate on an expansion side;
in which the link mechanism forming the second amplifying mechanism is provided in such an attitude that the link mechanism forming the first amplifying mechanism is altered 90° about a center axis parallel to an axis of pivot of each of the joints, and the fixed joint is fixed in position relative to an end of the piezoelectric elements laminate on a fixed side and the first movable joint is provided in a tip end of the second link in the link mechanism forming the first amplifying mechanism; and
in which the tip end of the second link in the link mechanism forming the second amplifying mechanism is rendered to be a displacement amplifying output unit of the link mechanism.

15. The microinjection apparatus as claimed in claim 9, in which the amplifying mechanism comprises a link mechanism and in which the link mechanism includes a cranking and sliding mechanism comprised of one fixed joint, two movable joint and two links, the cranking and sliding mechanism being capable of amplifying and converting a displacement of the piezoelectric elements in a direction of expansion thereof into an arbitrary direction along a circumference of the fixed joint through the two movable joints and the two links.

16. The microinjection apparatus as claimed in claim 15, in which the cranking and sliding mechanism is an offset cranking mechanism.

17. The microinjection apparatus as claimed in claim 7, in which the transfer mechanism for moving a needle support member, supporting the injection needle, in a direction conforming to a direction of insertion of the injection needle, is a transfer mechanism including the piezoelectric elements laminate as a drive source and includes a vibration driver for applying vibration to the needle support member in a direction conforming to the direction of insertion of the injection needle by vibrating a part of piezoelectric elements forming the transfer mechanism.

18. A microinjection method of introducing a transfecting material into a transductant by piercing a minute injection needle, filled with the transfecting material, into the transductant, which method comprises:
a container position adjusting step of adjusting a position of a container accommodating therein the transductant;
an imaging step of capturing a plan view image of an inside of the container, the position of which has been adjusted during the container position adjusting step, through a lens;
a position determining step of determining a position of the transductant from the image obtained during the imaging step; and
a step of individually moving each of a plurality of injection needles by means of a plurality of transporters according to a position information obtained during the position determining step.
